# EUROPÄISCHE PATENTANMELDUNG

(11) **EP 2 708 898 A1**
(43) Veröffentlichungstag der Anmeldung: **19.03.2014**
(21) Anmeldenummer: 12184500.2
(22) Anmeldetag: 14.09.2012
(51) Int. Cl.: G01N 33/68

(54) **Verfahren zur Diagnose eines molekularen Phänotyps eines an einer mit chronischen Entzündungen einhergehenden Erkrankung leidenden Patienten**

(71) Anmelder: Sterna Biologicals GmbH & Co. Kg, 35043 Marburg (DE)
(72) Erfinder: Bille, Joachim, Dr., 35447 Reiskirchen (DE); Turowska, Agnieszka, Dr., 35392 Gießen (DE)
(74) Vertreter: Patentanwälte Olbricht Buchhold Keulertz

(57) **Zusammenfassung**

Chronische Entzündungen stellen einen zunehmenden medizinischen Problemkreis von hoher sozioökonomischen Bedeutung dar. Die Erfindung betrifft ein Verfahren und ein Kit zur Diagnose eines molekularen Phänotyps eines an einer mit chronischen Entzündungen einhergehenden Erkrankung leidenden Patienten sowie ein Arzneimittel zur Behandlung eines solchen Patienten. Dazu wird die Genexpression von GATA-3 und/oder Tbet in einem biologischen Isolat des Patienten gemessen und für die Zuordnung zu einem molekularen Phänotyp der Erkrankung genutzt.

SEQUENCE LISTING

<110>  sterna biologicals GmbH & Co. KG

<120>  Verfahren zur Diagnose eines molekularen Phänotyps eines an einer
        chronischen Entzündungserkrankung leidenden humanen Patienten

<130>  TM661,80027-0061

<160>  155

<170>  PatentIn version 3.5

<210>  1
<211>  33
<212>  DNA
<213>  Artificial Sequence

<220>
<223>  DNAzyme

<220>
<221>  misc_feature
<222>  (1)..(33)
<223>  hgd1 DNAzyme against GATA-3mRNA

<400>  1
tcggtcagag gctagctaca acgatgcgtt gct                                    33

<210>  2
<211>  33
<212>  DNA
<213>  Artificial Sequence

<220>
<223>  DNAzyme against GATA-3 mRNA

<220>
<221>  misc_feature
<222>  (1)..(33)
<223>  hgd2 DNAzyme against GATA-3 mRNA

<400>  2
ggcgtacgag gctagctaca acgactgctc ggt                                    33

<210>  3
<211>  33
<212>  DNA
<213>  Artificial Sequence

<220>
<223>  DNAzyme

<220>
<221>  misc_feature
<222>  (1)..(33)
<223>  hgd3 DNAzyme against GATA-3 mRNA

<400>  3
ggcggcgtag gctagctaca acgagacctg ctc                                    33

<210>  4
<211>  33
<212>  DNA
<213>  Artificial Sequence

<220>
<223>  DNAzyme


<220>
<221>  misc_feature
<222>  (1)..(33)
<223>  hgd4 DNAzyme against GATA-3 mRNA

<400>  4
ctcgggtcag gctagctaca acgactgggt agc                                    33


<210>  5
<211>  33
<212>  DNA
<213>  Artificial Sequence

<220>
<223>  DNAzyme


<220>
<221>  misc_feature
<222>  (1)..(33)
<223>  hgd5 DNAzyme against GATA-3 mRNA

<400>  5
tcctctgcag gctagctaca acgacggggt cct                                    33


<210>  6
<211>  33
<212>  DNA
<213>  Artificial Sequence

<220>
<223>  DNAzyme


<220>
<221>  misc_feature
<222>  (1)..(33)
<223>  hgd6 DNAzyme against GATA-3 mRNA

<400>  6
actctgcaag gctagctaca acgatctgcg agc                                    33


<210>  7
<211>  33
<212>  DNA
<213>  Artificial Sequence

<220>
<223>  DNAzyme

<220>
<221>  misc_feature
<222>  (1)..(33)
<223>  hgd7 DNAzyme against GATA-3 mRNA

<400>  7
gggcgacgag gctagctaca acgatctgca att                                    33


<210>  8
<211>  33
<212>  DNA
<213>  Artificial Sequence

<220>
<223>  DNAzyme


<220>
<221>  misc_feature
<222>  (1)..(33)
<223>  hgd8 DNAzyme against GATA-3 mRNA

<400>  8
aaggggcgag gctagctaca acgagactct gca                                    33


<210>  9
<211>  33
<212>  DNA
<213>  Artificial Sequence

<220>
<223>  DNAzyme


<220>
<221>  misc_feature
<222>  (1)..(33)
<223>  hgd9 DNAzyme against GATA-3 mRNA

<400>  9
aaaacgggag gctagctaca acgacaggtt gta                                    33


<210>  10
<211>  33
<212>  DNA
<213>  Artificial Sequence

<220>
<223>  DNAzyme


<220>
<221>  misc_feature
<222>  (1)..(33)
<223>  hgd10 DNAzyme against GATA-3 mRNA

<400>  10
agaataaaag gctagctaca acgagggacc agg                                    33


<210>  11
<211>  33

<212>  DNA
<213>  Artificial Sequence

<220>
<223>  DNAzyme



<220>
<221>  misc_feature
<222>  (1)..(33)
<223>  hgd11 DNAzyme against GATA-3 mRNA

<400>  11
atggcagaag gctagctaca acgaaaaacg gga                                    33


<210>  12
<211>  33
<212>  DNA
<213>  Artificial Sequence

<220>
<223>  DNAzyme



<220>
<221>  misc_feature
<222>  (1)..(33)
<223>  hgd12 DNAzyme against GATA-3 mRNA

<400>  12
aactgggtag gctagctaca acgaggcaga ata                                    33


<210>  13
<211>  33
<212>  DNA
<213>  Artificial Sequence

<220>
<223>  DNAzyme



<220>
<221>  misc_feature
<222>  (1)..(33)
<223>  hgd13 DNAzyme against GATA-3 mRNA

<400>  13
atccaaaaag gctagctaca acgatgggta tgg                                    33


<210>  14
<211>  33
<212>  DNA
<213>  Artificial Sequence

<220>
<223>  DNAzyme



<220>
<221>  misc_feature
<222>  (1)..(33)
<223>  hgd14 DNAzyme against GATA-3 mRNA

<400>  14
aggggaagag gctagctaca acgaaaaaat cca                                    33


<210>  15
<211>  33
<212>  DNA
<213>  Artificial Sequence

<220>
<223>  hgd15 DNAzyme against GATA-3 mRNA


<220>
<221>  misc_feature
<222>  (1)..(33)
<223>  hgd15 DNAzyme against GATA-3 mRNA

<400>  15
ttttaaaaag gctagctaca acgatatctt gga                                    33


<210>  16
<211>  33
<212>  DNA
<213>  Artificial Sequence

<220>
<223>  DNAzyme


<220>
<221>  misc_feature
<222>  (1)..(33)
<223>  hgd16 DNAzyme against GATA-3 mRNA

<400>  16
gtggggggag gctagctaca acgagggaag gct                                    33


<210>  17
<211>  33
<212>  DNA
<213>  Artificial Sequence

<220>
<223>  DNAzyme


<220>
<221>  misc_feature
<222>  (1)..(33)
<223>  hgd17 DNAzyme against GATA-3 mRNA

<400>  17
gttgaatgag gctagctaca acgattgctt tcg                                    33


<210>  18
<211>  33
<212>  DNA
<213>  Artificial Sequence

<220>

<223>  DNAzyme

<220>
<221>  misc_feature
<222>  (1)..(33)
<223>  hgd18 DNAzyme against GATA-3 mRNA

<400>  18
gtcgttgaag gctagctaca acgagatttg ctt                                    33

<210>  19
<211>  33
<212>  DNA
<213>  Artificial Sequence

<220>
<223>  DNAzyme

<220>
<221>  misc_feature
<222>  (1)..(33)
<223>  hgd19 DNAzyme against GATA-3 mRNA

<400>  19
ggcccggaag gctagctaca acgaccgcgc gcg                                    33

<210>  20
<211>  33
<212>  DNA
<213>  Artificial Sequence

<220>
<223>  DNAzyme

<220>
<221>  misc_feature
<222>  (1)..(33)
<223>  hgd20 DNAzyme against GATA-3 mRNA

<400>  20
tcacctccag gctagctaca acgaggcctc ggc                                    33

<210>  21
<211>  33
<212>  DNA
<213>  Artificial Sequence

<220>
<223>  DNAzyme

<220>
<221>  misc_feature
<222>  (1)..(33)
<223>  hgd21 DNAzyme against GATA-3 mRNA

<400>  21
ccgccgtcag gctagctaca acgactccat ggc                                    33

<210>  22
<211>  33
<212>  DNA
<213>  Artificial Sequence

<220>
<223>  DNAzyme


<220>
<221>  misc_feature
<222>  (1)..(33)
<223>  hgd22 DNAzyme against GATA-3 mRNA

<400>  22
ggtggctcag gctagctaca acgaccagcg cgg                                33


<210>  23
<211>  33
<212>  DNA
<213>  Artificial Sequence

<220>
<223>  DNAzyme


<220>
<221>  misc_feature
<222>  (1)..(33)
<223>  hgd23 DNAzyme against GATA-3 mRNA

<400>  23
cgttgagcag gctagctaca acgaggcggg gtg                                33


<210>  24
<211>  33
<212>  DNA
<213>  Artificial Sequence

<220>
<223>  DNAzyme


<220>
<221>  misc_feature
<222>  (1)..(33)
<223>  hgd24 DNAzyme against GATA-3 mRNA

<400>  24
ccgcgtccag gctagctaca acgagtagga gtg                                33


<210>  25
<211>  33
<212>  DNA
<213>  Artificial Sequence

<220>
<223>  DNAzyme


<220>

<221>  misc_feature
<222>  (1)..(33)
<223>  hgd25 DNAzyme against GATA-3 mRNA

<400>  25
cagcgggtag gctagctaca acgatgcgcc gcg                                    33


<210>  26
<211>  33
<212>  DNA
<213>  Artificial Sequence

<220>
<223>  DNAzyme


<220>
<221>  misc_feature
<222>  (1)..(33)
<223>  hgd26 DNAzyme against GATA-3 mRNA

<400>  26
gcacatccag gctagctaca acgactcctc cgg                                    33


<210>  27
<211>  33
<212>  DNA
<213>  Artificial Sequence

<220>
<223>  DNAzyme


<220>
<221>  misc_feature
<222>  (1)..(33)
<223>  hgd27 DNAzyme against GATA-3 mRNA

<400>  27
aaaagcacag gctagctaca acgaccacct cct                                    33


<210>  28
<211>  33
<212>  DNA
<213>  Artificial Sequence

<220>
<223>  DNAzyme


<220>
<221>  misc_feature
<222>  (1)..(33)
<223>  hgd28 DNAzyme against GATA-3 mRNA

<400>  28
taaaaagcag gctagctaca acgaatccac ctc                                    33


<210>  29
<211>  33
<212>  DNA

<213>  Artificial Sequence

<220>
<223>  DNAzyme


<220>
<221>  misc_feature
<222>  (1)..(33)
<223>  hgd29 DNAzyme against GATA-3 mRNA

<400>  29
gaccgtcgag gctagctaca acgagttaaa aag                                    33


<210>  30
<211>  33
<212>  DNA
<213>  Artificial Sequence

<220>
<223>  DNAzyme


<220>
<221>  misc_feature
<222>  (1)..(33)
<223>  hgd30 DNAzyme against GATA-3 mRNA

<400>  30
ttgccttgag gctagctaca acgacgtcga tgt                                    33


<210>  31
<211>  33
<212>  DNA
<213>  Artificial Sequence

<220>
<223>  DNAzyme


<220>
<221>  misc_feature
<222>  (1)..(33)
<223>  hgd31 DNAzyme against GATA-3 mRNA

<400>  31
agggcgggag gctagctaca acgagtggtt gcc                                    33


<210>  32
<211>  33
<212>  DNA
<213>  Artificial Sequence

<220>
<223>  DNAzyme


<220>
<221>  misc_feature
<222>  (1)..(33)
<223>  hgd32 DNAzyme against GATA-3 mRNA

<400>  32
tggccctgag gctagctaca acgacgagtt tcc                                    33


<210>  33
<211>  33
<212>  DNA
<213>  Artificial Sequence

<220>
<223>  DNAzyme


<220>
<221>  misc_feature
<222>  (1)..(33)
<223>  hgd33 DNAzyme against GATA-3 mRNA

<400>  33
acctctgcag gctagctaca acgacgtggc cct                                    33


<210>  34
<211>  33
<212>  DNA
<213>  Artificial Sequence

<220>
<223>  DNAzyme


<220>
<221>  misc_feature
<222>  (1)..(33)
<223>  hgd34 DNAzyme against GATA-3 mRNA

<400>  34
cggagggtag gctagctaca acgactctgc acc                                    33


<210>  35
<211>  33
<212>  DNA
<213>  Artificial Sequence

<220>
<223>  DNAzyme


<220>
<221>  misc_feature
<222>  (1)..(33)
<223>  hgd35 DNAzyme against GATA-3 mRNA

<400>  35
ggcggcacag gctagctaca acgactggct ccc                                    33


<210>  36
<211>  33
<212>  DNA
<213>  Artificial Sequence

<220>
<223>  DNAzyme

<220>
<221>  misc_feature
<222>  (1)..(33)
<223>  hgd36 DNAzyme against GATA-3 mRNA

<400>  36
cgggcggcag gctagctaca acgaacctgg ctc                                    33


<210>  37
<211>  33
<212>  DNA
<213>  Artificial Sequence

<220>
<223>  DNAzyme


<220>
<221>  misc_feature
<222>  (1)..(33)
<223>  hgd37 DNAzyme against GATA-3mRNA

<400>  37
agggatccag gctagctaca acgagaagca gag                                    33


<210>  38
<211>  33
<212>  DNA
<213>  Artificial Sequence

<220>
<223>  DNAzyme


<220>
<221>  misc_feature
<222>  (1)..(33)
<223>  hgd38 DNAzyme against GATA-3 mRNA

<400>  38
gggtagggag gctagctaca acgaccatga agc                                    33


<210>  39
<211>  33
<212>  DNA
<213>  Artificial Sequence

<220>
<223>  DNAzyme


<220>
<221>  misc_feature
<222>  (1)..(33)
<223>  hgd39 DNAzyme against GATA-3 mRNA

<400>  39
gggctgagag gctagctaca acgatccagg ggg                                    33

<210>  40
<211>  33
<212>  DNA
<213>  Artificial Sequence

<220>
<223>  DNAzyme


<220>
<221>  misc_feature
<222>  (1)..(33)
<223>  hgd40 DNAzyme against GATA-3 mRNA

<400>  40
gtggatggag gctagctaca acgagtcttg gag                                    33


<210>  41
<211>  33
<212>  DNA
<213>  Artificial Sequence

<220>
<223>  DNAzyme


<220>
<221>  misc_feature
<222>  (1)..(33)
<223>  hgd41 DNAzyme against GATA-3 mRNA

<400>  41
cgtggtggag gctagctaca acgaggacgt ctt                                    33


<210>  42
<211>  33
<212>  DNA
<213>  Artificial Sequence

<220>
<223>  DNAzyme


<220>
<221>  misc_feature
<222>  (1)..(33)
<223>  hgd42 DNAzyme against GATA-3 mRNA

<400>  42
gggggtagag gctagctaca acgaggagag ggg                                    33


<210>  43
<211>  33
<212>  DNA
<213>  Artificial Sequence

<220>
<223>  DNAzyme


<220>
<221>  misc_feature

<222>  (1)..(33)
<223>  hgd43 DNAzyme against GATA-3 mRNA

<400>  43
ggaggaggag gctagctaca acgagaggcc ggg                                33


<210>  44
<211>  33
<212>  DNA
<213>  Artificial Sequence

<220>
<223>  DNAzyme


<220>
<221>  misc_feature
<222>  (1)..(33)
<223>  hgd44 DNAzyme against GATA-3 mRNA

<400>  44
gccccccgag gctagctaca acgaaaggag gag                                33


<210>  45
<211>  33
<212>  DNA
<213>  Artificial Sequence

<220>
<223>  DNAzyme


<220>
<221>  misc_feature
<222>  (1)..(33)
<223>  hgd45 DNAzyme against GATA-3 mRNA

<400>  45
ccggggagag gctagctaca acgagtcctt cgg                                33


<210>  46
<211>  33
<212>  DNA
<213>  Artificial Sequence

<220>
<223>  DNAzyme


<220>
<221>  misc_feature
<222>  (1)..(33)
<223>  hgd46 DNAzyme against GATA-3 mRNA

<400>  46
ggacagcgag gctagctaca acgagggtcc ggg                                33


<210>  47
<211>  33
<212>  DNA
<213>  Artificial Sequence

<220>
<223>  DNAzyme


<220>
<221>  misc_feature
<222>  (1)..(33)
<223>  hgd47 DNAzyme against GATA-3 mRNA

<400>  47
tggggtggag gctagctaca acgaagcgat ggg                                    33


<210>  48
<211>  33
<212>  DNA
<213>  Artificial Sequence

<220>
<223>  DNAzyme


<220>
<221>  misc_feature
<222>  (1)..(33)
<223>  hgd48 DNAzyme against GATA-3 mRNA

<400>  48
cttgaggcag gctagctaca acgatctttc tcg                                    33


<210>  49
<211>  33
<212>  DNA
<213>  Artificial Sequence

<220>
<223>  DNAzyme


<220>
<221>  misc_feature
<222>  (1)..(33)
<223>  hgd49 DNAzyme against GATA-3 mRNA

<400>  49
cacctggtag gctagctaca acgattgagg cac                                    33


<210>  50
<211>  33
<212>  DNA
<213>  Artificial Sequence

<220>
<223>  DNAzyme


<220>
<221>  misc_feature
<222>  (1)..(33)
<223>  hgd50 DNAzyme against GATA-3 mRNA

<400>  50

gcaggggcag gctagctaca acgactggta ctt                                    33

<210>  51
<211>  33
<212>  DNA
<213>  Artificial Sequence

<220>
<223>  DNAzyme

<220>
<221>  misc_feature
<222>  (1)..(33)
<223>  hgd51 DNAzyme against GATA-3 mRNA

<400>  51
ccagcttcag gctagctaca acgagctgtc ggg                                    33

<210>  52
<211>  33
<212>  DNA
<213>  Artificial Sequence

<220>
<223>  DNAzyme

<220>
<221>  misc_feature
<222>  (1)..(33)
<223>  hgd52 DNAzyme against GATA-3 mRNA

<400>  52
gtgggacgag gctagctaca acgatccagc ttc                                    33

<210>  53
<211>  33
<212>  DNA
<213>  Artificial Sequence

<220>
<223>  DNAzyme

<220>
<221>  misc_feature
<222>  (1)..(33)
<223>  hgd53 DNAzyme against GATA-3 mRNA

<400>  53
ggagtgggag gctagctaca acgagactcc agc                                    33

<210>  54
<211>  33
<212>  DNA
<213>  Artificial Sequence

<220>
<223>  DNAzyme

<220>
<221>  misc_feature
<222>  (1)..(33)
<223>  hgd54 DNAzyme against GATA-3 mRNA

<400>  54
atgctgccag gctagctaca acgagggagt ggg                                    33


<210>  55
<211>  33
<212>  DNA
<213>  Artificial Sequence

<220>
<223>  DNAzyme


<220>
<221>  misc_feature
<222>  (1)..(33)
<223>  hgd55 DNAzyme against GATA-3 mRNA

<400>  55
gggcggtcag gctagctaca acgagctgcc acg                                    33


<210>  56
<211>  33
<212>  DNA
<213>  Artificial Sequence

<220>
<223>  DNAzyme


<220>
<221>  misc_feature
<222>  (1)..(33)
<223>  hgd56 DNAzyme against GATA-3 mRNA

<400>  56
gaggctccag gctagctaca acgaccaggg cgg                                    33


<210>  57
<211>  33
<212>  DNA
<213>  Artificial Sequence

<220>
<223>  DNAzyme


<220>
<221>  misc_feature
<222>  (1)..(33)
<223>  hgd57 DNAzyme against GATA-3 mRNA

<400>  57
gtgggtcgag gctagctaca acgagaggag gct                                    33


<210>  58

<211>  33
<212>  DNA
<213>  Artificial Sequence

<220>
<223>  DNAzyme



<220>
<221>  misc_feature
<222>  (1)..(33)
<223>  hgd58 DNAzyme against GATA-3 mRNA

<400>  58
aggtggtgag gctagctaca acgaggggtg gtg                                    33


<210>  59
<211>  33
<212>  DNA
<213>  Artificial Sequence

<220>
<223>  DNAzyme



<220>
<221>  misc_feature
<222>  (1)..(33)
<223>  hgd59 DNAzyme against GATA-3 mRNA

<400>  59
actcgggcag gctagctaca acgagtaggg cgg                                    33


<210>  60
<211>  33
<212>  DNA
<213>  Artificial Sequence

<220>
<223>  DNAzyme



<220>
<221>  misc_feature
<222>  (1)..(33)
<223>  hgd60 DNAzyme against GATA-3 mRNA

<400>  60
ggagctgtag gctagctaca acgatcgggc acg                                    33


<210>  61
<211>  33
<212>  DNA
<213>  Artificial Sequence

<220>
<223>  DNAzyme



<220>
<221>  misc_feature
<222>  (1)..(33)

<223>  hgd61 DNAzyme against GATA-3 mRNA

<400>  61
ggacttgcag gctagctaca acgaccgaag ccg                                33


<210>  62
<211>  33
<212>  DNA
<213>  Artificial Sequence

<220>
<223>  DNAzyme


<220>
<221>  misc_feature
<222>  (1)..(33)
<223>  hgd62 DNAzyme against GATA-3 mRNA

<400>  62
gggcctggag gctagctaca acgattgcat ccg                                33


<210>  63
<211>  33
<212>  DNA
<213>  Artificial Sequence

<220>
<223>  DNAzyme


<220>
<221>  misc_feature
<222>  (1)..(33)
<223>  hgd63 DNAzyme against GATA-3 mRNA

<400>  63
tgtgctggag gctagctaca acgacgggcc ttg                                33


<210>  64
<211>  33
<212>  DNA
<213>  Artificial Sequence

<220>
<223>  DNAzyme


<220>
<221>  misc_feature
<222>  (1)..(33)
<223>  hgd64 DNAzyme against GATA-3 mRNA

<400>  64
gttcacacag gctagctaca acgatccctg cct                                33


<210>  65
<211>  33
<212>  DNA
<213>  Artificial Sequence

<220>
<223>  DNAzyme


<220>
<221>  misc_feature
<222>  (1)..(33)
<223>  hgd65 DNAzyme against GATA-3 mRNA

<400>  65
cagttcacag gctagctaca acgaactccc tgc                                    33


<210>  66
<211>  33
<212>  DNA
<213>  Artificial Sequence

<220>
<223>  DNAzyme


<220>
<221>  misc_feature
<222>  (1)..(33)
<223>  hgd66 DNAzyme against GATA-3 mRNA

<400>  66
cacagttcag gctagctaca acgaacactc cct                                    33


<210>  67
<211>  33
<212>  DNA
<213>  Artificial Sequence

<220>
<223>  DNAzyme


<220>
<221>  misc_feature
<222>  (1)..(33)
<223>  hgd67 DNAzyme against GATA-3 mRNA

<400>  67
gttgccccag gctagctaca acgaagttca cac                                    33


<210>  68
<211>  33
<212>  DNA
<213>  Artificial Sequence

<220>
<223>  DNAzyme


<220>
<221>  misc_feature
<222>  (1)..(33)
<223>  hgd68 DNAzyme against GATA-3 mRNA

<400>  68
tcgccgccag gctagctaca acgaagtggg gtc                                    33

<210>  69
<211>  33
<212>  DNA
<213>  Artificial Sequence

<220>
<223>  DNAzyme


<220>
<221>  misc_feature
<222>  (1)..(33)
<223>  hgd69 DNAzyme against GATA-3mRNA

<400>  69
cccgtgccag gctagctaca acgactcgcc gcc                                    33


<210>  70
<211>  33
<212>  DNA
<213>  Artificial Sequence

<220>
<223>  DNAzyme


<220>
<221>  misc_feature
<222>  (1)..(33)
<223>  hgd70 DNAzyme against GATA-3 mRNA

<400>  70
ggcgttgcag gctagctaca acgaaggtag tgt                                    33


<210>  71
<211>  33
<212>  DNA
<213>  Artificial Sequence

<220>
<223>  DNAzyme


<220>
<221>  misc_feature
<222>  (1)..(33)
<223>  td21 DNAzyme against T-bet mRNA

<400>  71
tggcttctag gctagctaca acgagccctc gtc                                    33


<210>  72
<211>  33
<212>  DNA
<213>  Artificial Sequence

<220>
<223>  DNAzyme

<220>
<221>  misc_feature
<222>  (1)..(33)
<223>  td2 DNAzyme against T-bet mRNA

<400>  72
gggctctgag gctagctaca acgagcctgg ctt                                    33


<210>  73
<211>  33
<212>  DNA
<213>  Artificial Sequence

<220>
<223>  DNAzyme


<220>
<221>  misc_feature
<222>  (1)..(33)
<223>  td3 DNAzyme against T-bet mRNA

<400>  73
gggaccccag gctagctaca acgacggagc ccg                                    33


<210>  74
<211>  33
<212>  DNA
<213>  Artificial Sequence

<220>
<223>  DNAzyme


<220>
<221>  misc_feature
<222>  (1)..(33)
<223>  td4 DNAzyme against T-bet mRNA

<400>  74
ggtgggggag gctagctaca acgacccacc gga                                    33


<210>  75
<211>  33
<212>  DNA
<213>  Artificial Sequence

<220>
<223>  DNAzyme


<220>
<221>  misc_feature
<222>  (1)..(33)
<223>  td5 DNAzyme against T-bet mRNA

<400>  75
ggcgggggag gctagctaca acgaccgagg gcc                                    33


<210>  76
<211>  33

<212>  DNA
<213>  Artificial Sequence

<220>
<223>  DNAzyme



<220>
<221>  misc_feature
<222>  (1)..(33)
<223>  td6 DNAzyme against T-bet mRNA

<400>  76
gggctgggag gctagctaca acgagggcag gga                                    33


<210>  77
<211>  33
<212>  DNA
<213>  Artificial Sequence

<220>
<223>  DNAzyme



<220>
<221>  misc_feature
<222>  (1)..(33)
<223>  td7 DNAzyme against T-bet mRNA

<400>  77
cgtcgaggag gctagctaca acgaccgccc ctc                                    33


<210>  78
<211>  33
<212>  DNA
<213>  Artificial Sequence

<220>
<223>  DNAzyme



<220>
<221>  misc_feature
<222>  (1)..(33)
<223>  td8 DNAzyme against T-bet mRNA

<400>  78
gggctggcag gctagctaca acgacttccc gta                                    33


<210>  79
<211>  33
<212>  DNA
<213>  Artificial Sequence

<220>
<223>  DNAzyme



<220>
<221>  misc_feature
<222>  (1)..(33)
<223>  td9 DNAzyme against T-bet mRNA

<400>  79
cgatgcccag gctagctaca acgaccgggg cgg                                    33


<210>  80
<211>  33
<212>  DNA
<213>  Artificial Sequence

<220>
<223>  DNAzyme


<220>
<221>  misc_feature
<222>  (1)..(33)
<223>  td10 DNAzyme against T-bet mRNA

<400>  80
gctccacgag gctagctaca acgagcccat ccg                                    33


<210>  81
<211>  33
<212>  DNA
<213>  Artificial Sequence

<220>
<223>  DNAzyme


<220>
<221>  misc_feature
<222>  (1)..(33)
<223>  td11 DNAzyme against T-bet mRNA

<400>  81
ccggctccag gctagctaca acgagatgcc cat                                    33


<210>  82
<211>  33
<212>  DNA
<213>  Artificial Sequence

<220>
<223>  DNAzyme


<220>
<221>  misc_feature
<222>  (1)..(33)
<223>  td12 DNAzyme against T-bet mRNA

<400>  82
tctccgcaag gctagctaca acgaccggct cca                                    33


<210>  83
<211>  33
<212>  DNA
<213>  Artificial Sequence

<220>

<223>  DNAzyme

<220>
<221>  misc_feature
<222>  (1)..(33)
<223>  td13 DNAzyme against T-bet mRNA

<400>  83
ccgtcagcag gctagctaca acgagtctcc gca                                    33

<210>  84
<211>  33
<212>  DNA
<213>  Artificial Sequence

<220>
<223>  DNAzyme

<220>
<221>  misc_feature
<222>  (1)..(33)
<223>  td14 DNAzyme against T-bet mRNA

<400>  84
tccccggcag gctagctaca acgacggctc ggt                                    33

<210>  85
<211>  33
<212>  DNA
<213>  Artificial Sequence

<220>
<223>  DNAzyme

<220>
<221>  misc_feature
<222>  (1)..(33)
<223>  td15 DNAzyme against T-bet mRNA

<400>  85
cccccgcgag gctagctaca acgagctcgt ccg                                    33

<210>  86
<211>  33
<212>  DNA
<213>  Artificial Sequence

<220>
<223>  DNAzyme

<220>
<221>  misc_feature
<222>  (1)..(33)
<223>  td16 DNAzyme against T-bet mRNA

<400>  86
gtagggagag gctagctaca acgacccagg ctg                                    33

<210>  87
<211>  33
<212>  DNA
<213>  Artificial Sequence

<220>
<223>  DNAzyme


<220>
<221>  misc_feature
<222>  (1)..(33)
<223>  td17 DNAzyme against T-bet mRNA

<400>  87
gggcgggcag gctagctaca acgacaaggc gcc                                    33


<210>  88
<211>  33
<212>  DNA
<213>  Artificial Sequence

<220>
<223>  DNAzyme


<220>
<221>  misc_feature
<222>  (1)..(33)
<223>  td18 DNAzyme against T-bet mRNA

<400>  88
cgggaaggag gctagctaca acgatcgccc gcg                                    33


<210>  89
<211>  33
<212>  DNA
<213>  Artificial Sequence

<220>
<223>  DNAzyme


<220>
<221>  misc_feature
<222>  (1)..(33)
<223>  td19 DNAzyme against T-bet mRNA

<400>  89
tagtcctcag gctagctaca acgagcggcc ccg                                    33


<210>  90
<211>  33
<212>  DNA
<213>  Artificial Sequence

<220>
<223>  DNAzyme


<220>

<221>  misc_feature
<222>  (1)..(33)
<223>  td20 DNAzyme against T-bet mRNA

<400>  90
tccccgacag gctagctaca acgactccag tcc                                    33


<210>  91
<211>  33
<212>  DNA
<213>  Artificial Sequence

<220>
<223>  DNAzyme


<220>
<221>  misc_feature
<222>  (1)..(33)
<223>  td21 DNAzyme against T-bet mRNA

<400>  91
tttccccgag gctagctaca acgaacctcc agt                                    33


<210>  92
<211>  33
<212>  DNA
<213>  Artificial Sequence

<220>
<223>  DNAzyme


<220>
<221>  misc_feature
<222>  (1)..(33)
<223>  td22 DNAzyme against T-bet mRNA

<400>  92
tgagcgcgag gctagctaca acgacctcag ttt                                    33


<210>  93
<211>  33
<212>  DNA
<213>  Artificial Sequence

<220>
<223>  DNAzyme


<220>
<221>  misc_feature
<222>  (1)..(33)
<223>  td23 DNAzyme against T-bet mRNA

<400>  93
ggaccacaag gctagctaca acgaaggtgg ttg                                    33


<210>  94
<211>  33
<212>  DNA

<213>  Artificial Sequence

<220>
<223>  DNAzyme



<220>
<221>  misc_feature
<222>  (1)..(33)
<223>  td24 DNAzyme against T-bet mRNA

<400>  94
cttggaccag gctagctaca acgaaacagg tgg                                    33


<210>  95
<211>  33
<212>  DNA
<213>  Artificial Sequence

<220>
<223>  DNAzyme



<220>
<221>  misc_feature
<222>  (1)..(33)
<223>  td25 DNAzyme against T-bet mRNA

<400>  95
aaacttggag gctagctaca acgacacaac agg                                    33


<210>  96
<211>  33
<212>  DNA
<213>  Artificial Sequence

<220>
<223>  DNAzyme



<220>
<221>  misc_feature
<222>  (1)..(33)
<223>  td26 DNAzyme against T-bet mRNA

<400>  96
ctgattaaag gctagctaca acgattggac cac                                    33


<210>  97
<211>  33
<212>  DNA
<213>  Artificial Sequence

<220>
<223>  DNAzyme



<220>
<221>  misc_feature
<222>  (1)..(33)
<223>  td27 DNAzyme against T-bet mRNA

<400>  97
tggtgctgag gctagctaca acgataaact tgg                                    33


<210>  98
<211>  33
<212>  DNA
<213>  Artificial Sequence

<220>
<223>  DNAzyme


<220>
<221>  misc_feature
<222>  (1)..(33)
<223>  td28 DNAzyme against T-bet mRNA

<400>  98
tgatgatcag gctagctaca acgactctgt ctg                                    33


<210>  99
<211>  33
<212>  DNA
<213>  Artificial Sequence

<220>
<223>  DNAzyme


<220>
<221>  misc_feature
<222>  (1)..(33)
<223>  td29 DNAzyme against T-bet mRNA

<400>  99
tggtgatgag gctagctaca acgacatctc tgt                                    33


<210>  100
<211>  33
<212>  DNA
<213>  Artificial Sequence

<220>
<223>  DNAzyme


<220>
<221>  misc_feature
<222>  (1)..(33)
<223>  td30 DNAzyme against T-bet mRNA

<400>  100
gcttggtgag gctagctaca acgagatcat ctc                                    33


<210>  101
<211>  33
<212>  DNA
<213>  Artificial Sequence

<220>
<223>  DNAzyme

<220>
<221>  misc_feature
<222>  (1)..(33)
<223>  td31 DNAzyme against T-bet mRNA

<400>  101
atgggaacag gctagctaca acgaccgccg tcc                                    33


<210>  102
<211>  33
<212>  DNA
<213>  Artificial Sequence

<220>
<223>  DNAzyme


<220>
<221>  misc_feature
<222>  (1)..(33)
<223>  td32 DNAzyme against T-bet mRNA

<400>  102
gaatgggaag gctagctaca acgaatccgc cgt                                    33


<210>  103
<211>  33
<212>  DNA
<213>  Artificial Sequence

<220>
<223>  DNAzyme


<220>
<221>  misc_feature
<222>  (1)..(33)
<223>  td33 DNAzyme against T-bet mRNA

<400>  103
tgacaggaag gctagctaca acgagggaac atc                                    33


<210>  104
<211>  33
<212>  DNA
<213>  Artificial Sequence

<220>
<223>  DNAzyme


<220>
<221>  misc_feature
<222>  (1)..(33)
<223>  td34 DNAzyme against T-bet mRNA

<400>  104
agtaaatgag gctagctaca acgaaggaat ggg                                    33

<210>  105
<211>  33
<212>  DNA
<213>  Artificial Sequence

<220>
<223>  DNAzyme


<220>
<221>  misc_feature
<222>  (1)..(33)
<223>  td35 DNAzyme against T-bet mRNA

<400>  105
cacagtaaag gctagctaca acgagacagg aat                                    33


<210>  106
<211>  33
<212>  DNA
<213>  Artificial Sequence

<220>
<223>  DNAzyme


<220>
<221>  misc_feature
<222>  (1)..(33)
<223>  td36 DNAzyme against T-bet mRNA

<400>  106
gcccggccag gctagctaca acgaagtaaa tga                                    33


<210>  107
<211>  33
<212>  DNA
<213>  Artificial Sequence

<220>
<223>  DNAzyme


<220>
<221>  misc_feature
<222>  (1)..(33)
<223>  td37 DNAzyme against T-bet mRNA

<400>  107
ccacaaacag gctagctaca acgacctgta gtg                                    33


<210>  108
<211>  33
<212>  DNA
<213>  Artificial Sequence

<220>
<223>  DNAzyme


<220>
<221>  misc_feature

<222>  (1)..(33)
<223>  td38 DNAzyme against T-bet mRNA

<400>  108
gtccacaaag gctagctaca acgaatcctg tag                                    33


<210>  109
<211>  33
<212>  DNA
<213>  Artificial Sequence

<220>
<223>  DNAzyme


<220>
<221>  misc_feature
<222>  (1)..(33)
<223>  td39 DNAzyme against T-bet mRNA

<400>  109
ccacgtccag gctagctaca acgaaaacat cct                                    33


<210>  110
<211>  33
<212>  DNA
<213>  Artificial Sequence

<220>
<223>  DNAzyme


<220>
<221>  misc_feature
<222>  (1)..(33)
<223>  td40 DNAzyme against T-bet mRNA

<400>  110
ccaagaccag gctagctaca acgagtccac aaa                                    33


<210>  111
<211>  33
<212>  DNA
<213>  Artificial Sequence

<220>
<223>  DNAzyme


<220>
<221>  misc_feature
<222>  (1)..(33)
<223>  td41 DNAzyme against T-bet mRNA

<400>  111
ccaccaagag gctagctaca acgacacgtc cac                                    33


<210>  112
<211>  33
<212>  DNA
<213>  Artificial Sequence

<220>
<223>  DNAzyme

<220>
<221>  misc_feature
<222>  (1)..(33)
<223>  td42 DNAzyme against T-bet mRNA

<400>  112
gctggtccag gctagctaca acgacaagac cac                                    33

<210>  113
<211>  33
<212>  DNA
<213>  Artificial Sequence

<220>
<223>  DNAzyme

<220>
<221>  misc_feature
<222>  (1)..(33)
<223>  td43 DNAzyme against T-bet mRNA

<400>  113
gctctggtag gctagctaca acgacgccag tgg                                    33

<210>  114
<211>  33
<212>  DNA
<213>  Artificial Sequence

<220>
<223>  DNAzyme

<220>
<221>  misc_feature
<222>  (1)..(33)
<223>  td44 DNAzyme against T-bet mRNA

<400>  114
ctgcacccag gctagctaca acgattgccg ctc                                    33

<210>  115
<211>  33
<212>  DNA
<213>  Artificial Sequence

<220>
<223>  DNAzyme

<220>
<221>  misc_feature
<222>  (1)..(33)
<223>  td45 DNAzyme against T-bet mRNA

<400>  115

cacactgcag gctagctaca acgaccactt gcc                                    33

<210>  116
<211>  33
<212>  DNA
<213>  Artificial Sequence

<220>
<223>  DNAzyme

<220>
<221>  misc_feature
<222>  (1)..(33)
<223>  td46 DNAzyme against T-bet mRNA

<400>  116
ctttccacag gctagctaca acgatgcacc cac                                    33

<210>  117
<211>  33
<212>  DNA
<213>  Artificial Sequence

<220>
<223>  DNAzyme

<220>
<221>  misc_feature
<222>  (1)..(33)
<223>  td47 DNAzyme against T-bet mRNA

<400>  117
gcctttccag gctagctaca acgaactgca ccc                                    33

<210>  118
<211>  33
<212>  DNA
<213>  Artificial Sequence

<220>
<223>  DNAzyme

<220>
<221>  misc_feature
<222>  (1)..(33)
<223>  td48 DNAzyme against T-bet mRNA

<400>  118
ttcctggcag gctagctaca acgagctgcc ctc                                    33

<210>  119
<211>  33
<212>  DNA
<213>  Artificial Sequence

<220>
<223>  DNAzyme

<220>
<221>  misc_feature
<222>  (1)..(33)
<223>  td49 DNAzyme against T-bet mRNA

<400>  119
gtggacgtag gctagctaca acgaaggcgg ttt                                    33


<210>  120
<211>  33
<212>  DNA
<213>  Artificial Sequence

<220>
<223>  DNAzyme


<220>
<221>  misc_feature
<222>  (1)..(33)
<223>  td50 DNAzyme against T-bet mRNA

<400>  120
ccgggtggag gctagctaca acgagtacag gcg                                    33


<210>  121
<211>  33
<212>  DNA
<213>  Artificial Sequence

<220>
<223>  DNAzyme


<220>
<221>  misc_feature
<222>  (1)..(33)
<223>  td51 DNAzyme against T-bet mRNA

<400>  121
cctggcgcag gctagctaca acgaccagtg cgc                                    33


<210>  122
<211>  33
<212>  DNA
<213>  Artificial Sequence

<220>
<223>  DNAzyme


<220>
<221>  misc_feature
<222>  (1)..(33)
<223>  td52 DNAzyme against T-bet mRNA

<400>  122
caaatgaaag gctagctaca acgattcctg gcg                                    33


<210>  123

<211>  33
<212>  DNA
<213>  Artificial Sequence

<220>
<223>  DNAzyme


<220>
<221>  misc_feature
<222>  (1)..(33)
<223>  td53 DNAzyme against T-bet mRNA

<400>  123
tttcccaaag gctagctaca acgagaaact tcc                                    33


<210>  124
<211>  33
<212>  DNA
<213>  Artificial Sequence

<220>
<223>  DNAzyme


<220>
<221>  misc_feature
<222>  (1)..(33)
<223>  td54 DNAzyme against T-bet mRNA

<400>  124
attgttggag gctagctaca acgagccccc ttg                                    33


<210>  125
<211>  33
<212>  DNA
<213>  Artificial Sequence

<220>
<223>  DNAzyme


<220>
<221>  misc_feature
<222>  (1)..(33)
<223>  td55 DNAzyme against T-bet mRNA

<400>  125
tgggtcacag gctagctaca acgatgttgg acg                                    33


<210>  126
<211>  33
<212>  DNA
<213>  Artificial Sequence

<220>
<223>  DNAzyme


<220>
<221>  misc_feature
<222>  (1)..(33)

<223>  td56 DNAzyme against T-bet mRNA

<400>  126
tctgggtcag gctagctaca acgaattgtt gga                                    33


<210>  127
<211>  33
<212>  DNA
<213>  Artificial Sequence

<220>
<223>  DNAzyme


<220>
<221>  misc_feature
<222>  (1)..(33)
<223>  td57 DNAzyme against T-bet mRNA

<400>  127
gcacaatcag gctagctaca acgactgggt cac                                    33


<210>  128
<211>  33
<212>  DNA
<213>  Artificial Sequence

<220>
<223>  DNAzyme


<220>
<221>  misc_feature
<222>  (1)..(33)
<223>  td58 DNAzyme against T-bet mRNA

<400>  128
ggagcacaag gctagctaca acgacatctg ggt                                    33


<210>  129
<211>  33
<212>  DNA
<213>  Artificial Sequence

<220>
<223>  DNAzyme


<220>
<221>  misc_feature
<222>  (1)..(33)
<223>  td59 DNAzyme against T-bet mRNA

<400>  129
actggagcag gctagctaca acgaaatcat ctg                                    33


<210>  130
<211>  33
<212>  DNA
<213>  Artificial Sequence

<220>
<223>  DNAzyme


<220>
<221>  misc_feature
<222>  (1)..(33)
<223>  td60 DNAzyme against T-bet mRNA

<400>  130
atggagggag gctagctaca acgatggagc aca                                    33


<210>  131
<211>  33
<212>  DNA
<213>  Artificial Sequence

<220>
<223>  DNAzyme


<220>
<221>  misc_feature
<222>  (1)..(33)
<223>  td61 DNAzyme against T-bet mRNA

<400>  131
tggtacttag gctagctaca acgaggaggg act                                    33


<210>  132
<211>  33
<212>  DNA
<213>  Artificial Sequence

<220>
<223>  DNAzyme


<220>
<221>  misc_feature
<222>  (1)..(33)
<223>  td62 DNAzyme against T-bet mRNA

<400>  132
gggctggtag gctagctaca acgattatgg agg                                    33


<210>  133
<211>  33
<212>  DNA
<213>  Artificial Sequence

<220>
<223>  DNAzyme


<220>
<221>  misc_feature
<222>  (1)..(33)
<223>  td63 DNAzyme against T-bet mRNA

<400>  133
tcaacgatag gctagctaca acgagcagcc ggg                                    33

<210>  134
<211>  33
<212>  DNA
<213>  Artificial Sequence

<220>
<223>  DNAzyme


<220>
<221>  misc_feature
<222>  (1)..(33)
<223>  td64 DNAzyme against T-bet mRNA

<400>  134
cctcaacgag gctagctaca acgaatgcag ccg                                    33


<210>  135
<211>  33
<212>  DNA
<213>  Artificial Sequence

<220>
<223>  DNAzyme


<220>
<221>  misc_feature
<222>  (1)..(33)
<223>  td65 DNAzyme against T-bet mRNA

<400>  135
tcacctcaag gctagctaca acgagatatg cag                                    33


<210>  136
<211>  33
<212>  DNA
<213>  Artificial Sequence

<220>
<223>  DNAzyme


<220>
<221>  misc_feature
<222>  (1)..(33)
<223>  td66 DNAzyme against T-bet mRNA

<400>  136
cgtcgttcag gctagctaca acgactcaac gat                                    33


<210>  137
<211>  33
<212>  DNA
<213>  Artificial Sequence

<220>
<223>  DNAzyme

<220>
<221>  misc_feature
<222>  (1)..(33)
<223>  td67 DNAzyme against T-bet mRNA

<400>  137
gtaaagatag gctagctaca acgagcgtgt tgg                                33


<210>  138
<211>  33
<212>  DNA
<213>  Artificial Sequence

<220>
<223>  DNAzyme


<220>
<221>  misc_feature
<222>  (1)..(33)
<223>  td68 DNAzyme against T-bet mRNA

<400>  138
aagtaaagag gctagctaca acgaatgcgt gtt                                33


<210>  139
<211>  33
<212>  DNA
<213>  Artificial Sequence

<220>
<223>  DNAzyme


<220>
<221>  misc_feature
<222>  (1)..(33)
<223>  td69 DNAzyme against T-bet mRNA

<400>  139
ggcaatgaag gctagctaca acgatgggtt tct                                33


<210>  140
<211>  33
<212>  DNA
<213>  Artificial Sequence

<220>
<223>  DNAzyme


<220>
<221>  misc_feature
<222>  (1)..(33)
<223>  td70 DNAzyme against T-bet mRNA

<400>  140
tcacggcaag gctagctaca acgagaactg ggt                                33


<210>  141
<211>  33

<212>  DNA
<213>  Artificial Sequence

<220>
<223>  DNAzyme



<220>
<221>  misc_feature
<222>  (1)..(33)
<223>  td71 DNAzyme against T-bet mRNA

<400>  141
aggcagtcag gctagctaca acgaggcaat gaa                                    33


<210>  142
<211>  33
<212>  DNA
<213>  Artificial Sequence

<220>
<223>  DNAzyme



<220>
<221>  misc_feature
<222>  (1)..(33)
<223>  td72 DNAzyme against T-bet mRNA

<400>  142
atctcggcag gctagctaca acgatctggt agg                                    33


<210>  143
<211>  33
<212>  DNA
<213>  Artificial Sequence

<220>
<223>  DNAzyme



<220>
<221>  misc_feature
<222>  (1)..(33)
<223>  td73 DNAzyme against T-bet mRNA

<400>  143
gctgagtaag gctagctaca acgactcggc att                                    33


<210>  144
<211>  33
<212>  DNA
<213>  Artificial Sequence

<220>
<223>  DNAzyme



<220>
<221>  misc_feature
<222>  (1)..(33)
<223>  td74 DNAzyme against T-bet mRNA

<400>  144
tattatcaag gctagctaca acgatttcag ctg                                    33


<210>  145
<211>  33
<212>  DNA
<213>  Artificial Sequence

<220>
<223>  DNAzyme



<220>
<221>  misc_feature
<222>  (1)..(33)
<223>  td75 DNAzyme against T-bet mRNA

<400>  145
gggttattag gctagctaca acgacaattt tca                                    33


<210>  146
<211>  33
<212>  DNA
<213>  Artificial Sequence

<220>
<223>  DNAzyme



<220>
<221>  misc_feature
<222>  (1)..(33)
<223>  td76 DNAzyme against T-bet mRNA

<400>  146
aaggggttag gctagctaca acgatatcaa ttt                                    33


<210>  147
<211>  33
<212>  DNA
<213>  Artificial Sequence

<220>
<223>  DNAzyme



<220>
<221>  misc_feature
<222>  (1)..(33)
<223>  td77 DNAzyme against T-bet mRNA

<400>  147
ctcccggaag gctagctaca acgacctttg gca                                    33


<210>  148
<211>  33
<212>  DNA
<213>  Artificial Sequence

<220>

<223>  DNAzyme

<220>
<221>  misc_feature
<222>  (1)..(33)
<223>  td78 DNAzyme against T-bet mRNA

<400>  148
gtacatggag gctagctaca acgatcaaag ttc                                    33

<210>  149
<211>  2588
<212>  DNA
<213>  Homo sapiens

<220>
<221>  gene
<222>  (1)..(2588)
<223>  Seq1 T-bet

<220>
<221>  misc_feature
<222>  (952)..(970)
<223>  td54 bindingsite

<220>
<221>  misc_feature
<222>  (1096)..(1114)
<223>  td69 bindingsite

<220>
<221>  misc_feature
<222>  (1100)..(1118)
<223>  td70 bindingsite

<400>  149
   cggcccgctg gagaggaagc ccgagagctg ccgcgcgcct gccggacgag ggcgtagaag      60
   ccaggcgtca gagcccgggc tccggtgggg tcccccaccc ggccctcggg tcccccgccc     120
   cctgctccct gcccatccca gcccacgcga ccctctcgcg cgcggagggg cgggtcctcg     180
   acggctacgg gaaggtgcca gcccgccccg gatgggcatc gtggagccgg gttgcggaga     240
   catgctgacg ggcaccgagc cgatgccggg gagcgacgag ggccgggcgc ctggcgccga     300
   cccgcagcac cgctacttct acccggagcc gggcgcgcag gacgcggacg agcgtcgcgg     360
   gggcggcagc ctggggtctc cctacccggg gggcgccttg gtgcccgccc cgccgagccg     420
   cttccttgga gcctacgcct acccgccgcg accccaggcg gccggcttcc ccggcgcggg     480
   cgagtccttc ccgccgcccg cggacgccga gggctaccag ccgggcgagg gctacgccgc     540
   cccggacccg cgcgccgggc tctacccggg gccgcgtgag gactacgcgc tacccgcggg     600
   actggaggtg tcggggaaac tgagggtcgc gctcaacaac cacctgttgt ggtccaagtt     660
   taatcagcac cagacagaga tgatcatcac caagcaggga cggcggatgt tcccattcct     720
   gtcatttact gtggccgggc tggagcccac cagccactac aggatgtttg tggacgtggt     780
   cttggtggac cagcaccact ggcggtacca gagcggcaag tgggtgcagt gtggaaaggc 840
   cgagggcagc atgccaggaa accgcctgta cgtccacccg gactccccca acacaggagc 900
   gcactggatg cgccaggaag tttcatttgg gaaactaaag ctcacaaaca acaagggggc 960
   gtccaacaat gtgacccaga tgattgtgct ccagtccctc cataagtacc agccccggct 1020
   gcatatcgtt gaggtgaacg acggagagcc agaggcagcc tgcaacgctt ccaacacgca 1080
   tatctttact ttccaagaaa cccagttcat tgccgtgact gcctaccaga atgccgagat 1140
   tactcagctg aaaattgata ataacccctt tgccaaagga ttccgggaga actttgagtc 1200
   catgtacaca tctgttgaca ccagcatccc ctccccgcct ggacccaact gtcaattcct 1260
   tgggggagat cactactctc ctctcctacc caaccagtat cctgttccca gccgcttcta 1320
   ccccgacctt cctggccagg cgaaggatgt ggttccccag gcttactggc tgggggcccc 1380
   ccgggaccac agctatgagg ctgagtttcg agcagtcagc atgaagcctg cattcttgcc 1440
   ctctgcccct gggcccacca tgtcctacta ccgaggccag gaggtcctgg cacctggagc 1500
   tggctggcct gtggcacccc agtaccctcc caagatgggc ccggccagct ggttccgccc 1560
   tatgcggact ctgcccatgg aacccggccc tggaggctca gagggacggg gaccagagga 1620
   ccagggtccc cccttggtgt ggactgagat tgcccccatc cggccggaat ccagtgattc 1680
   aggactgggc gaaggagact ctaagaggag gcgcgtgtcc ccctatcctt ccagtggtga 1740
   cagctcctcc cctgctgggg ccccttctcc ttttgataag gaagctgaag gacagtttta 1800
   taactatttt cccaactgag cagatgacat gatgaaagga acagaaacag tgttattagg 1860
   ttggaggaca ccgactaatt tgggaaacgg atgaaggact gagaaggccc ccgctccctc 1920
   tggcccttct ctgtttagta gttggttggg gaagtggggc tcaagaagga ttttggggtt 1980
   caccagatgc ttcctggccc acgatgaaac ctgagagggg tgtccccttg ccccatcctc 2040
   tgccctaact acagtcgttt acctggtgct gcgtcttgct tttggtttcc agctggagaa 2100
   aagaagacaa gaaagtcttg ggcatgaagg agctttttgc atctagtggg tgggaggggt 2160
   caggtgtggg acatgggagc aggagactcc actttcttcc tttgtacagt aactttcaac 2220
   cttttcgttg gcatgtgtgt taatccctga tccaaaaaga acaaatacac gtatgttata 2280
   accatcagcc cgccagggtc agggaaagga ctcacctgac tttggacagc tggcctgggc 2340
   tccccctgct caaacacagt ggggatcaga gaaaaggggc tggaaagggg ggaatggccc 2400
   acatctcaag aagcaagata ttgtttgtgg tggttgtgtg tgggtgtgtg ttttttcttt 2460
   ttctttcttt ttattttttt tgaatggggg aggctattta ttgtactgag agtggtgtct 2520
   ggatatattc cttttgtctt catcactttc tgaaataaac ataaaactgt taaaaaaaaa 2580
   aaaaaaaa 2588

<210> 150
<211> 2450

<212>  DNA
<213>  Homo sapiens

<220>
<221>  gene
<222>  (1)..(2450)
<223>  Seq2 T-bet

<220>
<221>  mutation
<222>  (134)..(134)
<223>  mutation

<220>
<221>  mutation
<222>  (310)..(310)
<223>  mutation

<220>
<221>  misc_feature
<222>  (952)..(970)
<223>  td54 bindingsite

<220>
<221>  misc_feature
<222>  (1096)..(1114)
<223>  td69 bindingsite

<220>
<221>  misc_feature
<222>  (1100)..(1118)
<223>  td70 bindingsite

<220>
<221>  mutation
<222>  (1399)..(1399)
<223>  mutation

<220>
<221>  mutation
<222>  (1556)..(1556)
<223>  mutation

<400>  150
cggcccgctg gagaggaagc ccgagagctg ccgcgcgcct gccggacgag ggcgtagaag      60

ccaggcgtca gagcccgggc tccggtgggg tcccccaccc ggccctcggg tcccccgccc     120

cctgctccct gcctatccca gcccacgcga ccctctcgcg cgcggagggg cgggtcctcg     180

acggctacgg gaaggtgcca gcccgccccg gatgggcatc gtggagccgg gttgcggaga     240

catgctgacg ggcaccgagc cgatgccggg gagcgacgag ggccgggcgc ctggcgccga     300

cccgcagcag cgctacttct acccggagcc gggcgcgcag gacgcggacg agcgtcgcgg     360

gggcggcagc ctggggtctc cctacccggg gggcgccttg gtgcccgccc cgccgagccg     420

cttccttgga gcctacgcct acccgccgcg accccaggcg gccggcttcc ccggcgcggg     480

cgagtccttc ccgccgcccg cggacgccga gggctaccag ccgggcgagg gctacgccgc     540

cccggacccg cgcgccgggc tctacccggg gccgcgtgag gactacgcgc tacccgcggg     600

actggaggtg tcggggaaac tgagggtcgc gctcaacaac cacctgttgt ggtccaagtt 660

taatcagcac cagacagaga tgatcatcac caagcaggga cggcggatgt tcccattcct 720

gtcatttact gtggccgggc tggagcccac cagccactac aggatgtttg tggacgtggt 780

cttggtggac cagcaccact ggcggtacca gagcggcaag tgggtgcagt gtggaaaggc 840

cgagggcagc atgccaggaa accgcctgta cgtccacccg gactccccca acacaggagc 900

gcactggatg cgccaggaag tttcatttgg gaaactaaag ctcacaaaca acaagggggc 960

gtccaacaat gtgacccaga tgattgtgct ccagtccctc cataagtacc agccccggct 1020

gcatatcgtt gaggtgaacg acggagagcc agaggcagcc tgcaacgctt ccaacacgca 1080

tatctttact ttccaagaaa cccagttcat tgccgtgact gcctaccaga atgccgagat 1140

tactcagctg aaaattgata ataacccctt tgccaaagga ttccgggaga actttgagtc 1200

catgtacaca tctgttgaca ccagcatccc ctccccgcct ggacccaact gtcaattcct 1260

tgggggagat cactactctc ctctcctacc caaccagtat cctgttccca gccgcttcta 1320

ccccgacctt cctggccagg cgaaggatgt ggttccccag gcttactggc tgggggcccc 1380

ccgggaccac agctatgggg ctgagtttcg agcagtcagc atgaagcctg cattcttgcc 1440

ctctgcccct gggcccacca tgtcctacta ccgaggccag gaggtcctgg cacctggagc 1500

tggctggcct gtggcacccc agtaccctcc caagatgggc ccggccagct ggttcagccc 1560

tatgcggact ctgcccatgg aacccggccc tggaggctca gagggacggg gaccagagga 1620

ccagggtccc cccttggtgt ggactgagat tgcccccatc cggccggaat ccagtgattc 1680

aggactgggc gaaggagact ctaagaggag gcgcgtgtcc ccctatcctt ccagtggtga 1740

cagctcctcc cctgctgggg ccccttctcc ttttgataag gaagctgaag gacagtttta 1800

taactatttt cccaactgag cagatgacat gatgaaagga acagaaacag tgttattagg 1860

ttggaggaca ccgactaatt tgggaaacgg atgaaggact gagaaggccc ccgctccctc 1920

tggcccttct ctgtttagta gttggttggg gaagtggggc tcaagaagga ttttggggtt 1980

caccagatgc ttcctggccc acgatgaaac ctgagagggg tgtccccttg ccccatcctc 2040

tgccctaact acagtcgttt acctggtgct gcgtcttgct tttggtttcc agctggagaa 2100

aagaagacaa gaaagtcttg ggcatgaagg agctttttgc atctagtggg tgggaggggt 2160

caggtgtggg acatgggagc aggagactcc actttcttcc tttgtacagt aactttcaac 2220

cttttcgttg gcatgtgtgt taatccctga tccaaaaaga acaaatacac gtatgttata 2280

accatcagcc cgccagggtc agggaaagga ctcacctgac tttggacagc tggcctgggc 2340

tccccctgct caaacacagt ggggatcaga gaaaaggggc tggaaagggg ggaatggccc 2400

acatctcaag aagcaagata ttgtttgtgg tggttgtgtg tgggtgtgtg 2450

<210> 151
<211> 2399

<212>  DNA
<213>  Homo sapiens

<220>
<221>  gene
<222>  (1)..(2399)
<223>  Sequenz_1 GATA-3

<400>  151
ggcgccgtct tgatactttc agaaagaatg cattccctgt aaaaaaaaaa aaaaaatact      60

gagagaggga gagagagaga gaagaagaga gagagacgga gggagagcga gacagagcga     120

gcaacgcaat ctgaccgagc aggtcgtacg ccgccgcctc ctcctcctct ctgctcttcg     180

ctacccaggt gacccgagga gggactccgc ctccgagcgg ctgaggaccc cggtgcagag     240

gagcctggct cgcagaattg cagagtcgtc gccccttttt acaacctggt cccgttttat     300

tctgccgtac ccagtttttg gatttttgtc ttccccttct tctctttgct aaacgacccc     360

tccaagataa tttttaaaaa accttctcct ttgctcacct ttgcttccca gccttcccat     420

ccccccaccg aaagcaaatc attcaacgac ccccgaccct ccgacggcag gagccccccg     480

acctcccagg cggaccgccc tccctccccg cgcgcgggtt ccgggcccgg cgagagggcg     540

cgagcacagc cgaggccatg gaggtgacgg cggaccagcc gcgctgggtg agccaccacc     600

accccgccgt gctcaacggg cagcacccgg acacgcacca cccgggcctc agccactcct     660

acatggacgc ggcgcagtac ccgctgccgg aggaggtgga tgtgcttttt aacatcgacg     720

gtcaaggcaa ccacgtcccg ccctactacg gaaactcggt cagggccacg gtgcagaggt     780

accctccgac ccaccacggg agccaggtgt gccgcccgcc tctgcttcat ggatccctac     840

cctggctgga cggcggcaaa gccctgggca gccaccacac cgcctccccc tggaatctca     900

gccccttctc caagacgtcc atccaccacg gctccccggg gcccctctcc gtctaccccc     960

cggcctcgtc ctcctccttg tcgggggggc acgccagccc gcacctcttc accttcccgc    1020

ccaccccgcc gaaggacgtc tccccggacc catcgctgtc caccccaggc tcggccggct    1080

cggcccggca ggacgagaaa gagtgcctca agtaccaggt gcccctgccc gacagcatga    1140

agctggagtc gtcccactcc cgtggcagca tgaccgccct gggtggagcc tcctcgtcga    1200

cccaccaccc catcaccacc tacccgccct acgtgcccga gtacagctcc ggactcttcc    1260

cccccagcag cctgctgggc ggctccccca ccggcttcgg atgcaagtcc aggcccaagg    1320

cccggtccag cacagaaggc agggagtgtg tgaactgtgg ggcaacctcg accccactgt    1380

ggcggcgaga tggcacggga cactacctgt gcaacgcctg cgggctctat cacaaaatga    1440

acggacagaa ccggcccctc attaagccca agcgaaggct gtctgcagcc aggagagcag    1500

ggacgtcctg tgcgaactgt cagaccacca caaccacact ctggaggagg aatgccaatg    1560

gggaccctgt ctgcaatgcc tgtgggctct actacaagct tcacaatatt aacagacccc    1620

tgactatgaa gaaggaaggc atccagacca gaaaccgaaa aatgtctagc aaatccaaaa    1680

agtgcaaaaa agtgcatgac tcactggagg acttccccaa gaacagctcg tttaacccgg 1740

ccgccctctc cagacacatg tcctccctga gccacatctc gcccttcagc cactccagcc 1800

acatgctgac cacgcccacg ccgatgcacc cgccatccag cctgtccttt ggaccacacc 1860

acccctccag catggtcacc gccatgggtt agagccctgc tcgatgctca cagggccccc 1920

agcgagagtc cctgcagtcc ctttcgactt gcatttttgc aggagcagta tcatgaagcc 1980

taaacgcgat ggatatatgt ttttgaaggc agaaagcaaa attatgtttg ccactttgca 2040

aaggagctca ctgtggtgtc tgtgttccaa ccactgaatc tggaccccat ctgtgaataa 2100

gccattctga ctcatatccc ctatttaaca gggtctctag tgctgtgaaa aaaaaaatgc 2160

tgaacattgc atataactta tattgtaaga aatactgtac aatgacttta ttgcatctgg 2220

gtagctgtaa ggcatgaagg atgccaagaa gtttaaggaa tatgggagaa atagtgtgga 2280

aattaagaag aaactaggtc tgatattcaa atggacaaac tgccagtttt gtttcctttc 2340

actggccaca gttgtttgat gcattaaaag aaaataaaaa aaagaaaaaa gagaaaaga 2399

<210> 152
<211> 2365
<212> DNA
<213> Homo sapiens

<220>
<221> gene
<222> (1)..(2365)
<223> Sequenz_2 GATA-3

<400> 152
tcccagcctt cccatccccc caccgaaagc aaatcattca acgacccccg accctccgac 60

ggcaggagcc ccccgacctc ccaggcggac cgcccttccc tccccgcgcg ggttccgggc 120

ccggcgagag ggcgcgacga cagccgaggc catggaggtg acggcggacc agccgcgctg 180

ggtgagccac caccaccccg ccgtgctcaa cgggcagcac ccggacacgc accacccggg 240

cctcagccac tcctacatgg acgcggcgca gtacccgctg ccggaggagg tggatgtgct 300

ttttaacatc gacggtcaag gcaaccacgt cccgccctac tacggaaact cggtcagggc 360

cacggtgcag aggtaccctc cgacccacca cgggagccag gtgtgccgcc cgcctctgct 420

tcatggatcc ctaccctggc tggacggcgg caaagccctg ggcagccacc acaccgcctc 480

cccctggaat ctcagcccct tctccaagac gtccatccac cacggctccc cggggcccct 540

ctccgtctac cccccggcct cgtcctcctc cttgtcgggg ggccacgcca gcccgcacct 600

cttcaccttc ccgcccaccc cgccgaagga cgtctccccg gacccatcgc tgtccacccc 660

aggctcggcc ggctcggccc ggcaggacga gaaagagtgc ctcaagtacc aggtgcccct 720

gcccgacagc atgaagctgg agtcgtccca ctcccgtggc agcatgaccg ccctgggtgg 780

agcctcctcg tcgacccacc accccatcac cacctacccg ccctacgtgc ccgagtacag 840

ctccggactc ttccccccca gcagcctgct gggcggctcc cccaccggct tcggatgcaa 900

gtccaggccc aaggcccggt ccagcacagg cagggagtgt gtgaactgtg gggcaacctc 960

gaccccactg tggcggcgag atggcacggg acactacctg tgcaacgcct gcgggctcta 1020

tcacaaaatg aacggacaga accggcccct cattaagccc aagcgaaggc tgtctgcagc 1080

caggagagca gggacgtcct gtgcgaactg tcagaccacc acaaccacac tctggaggag 1140

gaatgccaat ggggaccctg tctgcaatgc ctgtgggctc tactacaagc ttcacaatat 1200

taacagaccc ctgactatga agaaggaagg catccagacc agaaaccgaa aaatgtctag 1260

caaatccaaa aagtgcaaaa aagtgcatga ctcactggag gacttcccca agaacagctc 1320

gtttaacccg gccgccctct ccagacacat gtcctccctg agccacatct cgcccttcag 1380

ccactccagc cacatgctga ccacgcccac gccgatgcac ccgccatcca gcctgtcctt 1440

tggaccacac cacccctcca gcatggtcac cgccatgggt tagagccctg ctcgatgctc 1500

acagggcccc cagcgagagt ccctgcagtc cctttcgact tgcatttttg caggagcagt 1560

atcatgaagc ctaaacgcga tggatatatg tttttgaagg cagaaagcaa aattatgttt 1620

gccactttgc aaaggagctc actgtggtgt ctgtgttcca accactgaat ctggacccca 1680

tctgtgaata agccattctg actcatatcc cctatttaac agggtctcta gtgctgtgaa 1740

aaaaaaaaat cctgaacatt gcatataact tatattgtaa gaaatactgt acaatgactt 1800

tattgcatct gggtagctgt aaggcatgaa ggatgccaag aagtttaagg aatatgggag 1860

aaatagtgtg gaaattaaga agaaactagg tctgatattc aaatggacaa actgccagtt 1920

ttgtttcctt tcactggcca cagttgtttg atgcattaaa agaaaataaa aaaaagaaaa 1980

aagagaaaag aaaaaaaaag aaaaaagttg taggcgaatc atttgttcaa agctgttggc 2040

cctctgcaaa ggaaatacca gttctgggca atcagtgtta ccgttcacca gttgccattg 2100

agggtttcag agagcctttt tctaggccta catgctttgt gaacaagtcc ctgtaattgt 2160

tgtttgtatg tataattcaa agcaccaaaa taagaaaaga tgtagattta tttcatcata 2220

ttatacagac cgaactgttg tataaattta tttactgcta gtcttaagaa ctgctttctt 2280

tcgtttgttt gtttcaatat tttccttctc tctcaatttt cggttgaata aactagatta 2340

cattcagttg gcaaaaaaaa aaaaa 2365

<210> 153
<211> 2728
<212> DNA
<213> Homo sapiens

<220>
<221> gene
<222> (1)..(2728)
<223> Sequenz_3 GATA-3

<220>
<221>  mutation
<222>  (57)..(57)
<223>  mutation

<220>
<221>  mutation
<222>  (59)..(59)
<223>  mutation

<220>
<221>  mutation
<222>  (69)..(69)
<223>  mutation

<220>
<221>  misc_feature
<222>  (909)..(927)
<223>  hgd40 bindingsite

<400>  153
   ggcgccgtct tgatactttc agaaagaatg cattccctgt aaaaaaaaaa aaaaaaaaat      60
   actgagagag ggagagagag agaagaagag agagagacgg agggagagcg agacagagcg     120
   agcaacgcaa tctgaccgag caggtcgtac gccgccgcct cctcctcctc tctgctcttc     180
   gctacccagg tgacccgagg agggactccg cctccgagcg gctgaggacc ccggtgcaga     240
   ggagcctggc tcgcagaatt gcagagtcgt cgcccctttt tacaacctgg tcccgtttta     300
   ttctgccata cccagttttt ggatttttgt cttccccttc ttctctttgc taaacgaccc     360
   ctccaagata atttttaaaa aaccttctcc tttgctcacc tttgcttccc agccttccca     420
   tccccccacc gaaagcaaat cattcaacga cccccgaccc tccgacggca ggagcccccc     480
   gacctcccag gcggaccgcc ctccctcccc gcgcgcgggt tccgggcccg gcgagagggc     540
   gcgagcacag ccgaggccat ggaggtgacg gcggaccagc cgcgctgggt gagccaccac     600
   caccccgccg tgctcaacgg gcagcacccg gacacgcacc acccgggcct cagccactcc     660
   tacatggacg cggcgcagta cccgctgccg gaggaggtgg atgtgctttt taacatcgac     720
   ggtcaaggca accacgtccc gccctactac ggaaactcgg tcagggccac ggtgcagagg     780
   taccctccga cccaccacgg gagccaggtg tgccgcccgc ctctgcttca tggatccctc     840
   cctggctgga cggcggcaaa gccctgggca gccaccacac cgcctccccc tggaatctca     900
   gccccttctc caagacgtcc atccaccacg gctccccggg gcccctctcc gtctaccccc     960
   cggcctcgtc ctcctccttg tcggggggcc acgccagccc gcacctcttc accttcccgc    1020
   ccaccccgcc gaaggacgtc tccccggacc catcgctgtc caccccaggc tcggccggct    1080
   cggcccggca ggacgagaaa gagtgcctca agtaccaggt gcccctgccc gacagcatga    1140
   agctggagtc gtcccactcc cgtggcagca tgaccgccct gggtggagcc tcctcgtcga    1200
   cccaccaccc catcaccacc tacccgccct acgtgcccga gtacagctcc ggactcttcc    1260
   cccccagcag cctgctgggc ggctccccca ccggcttcgg atgcaagtcc aggcccaagg    1320
   cccggtccag cacagaaggc agggagtgtg tgaactgtgg ggcaacctcg accccactgt 1380
   ggcggcgaga tggcacggga cactacctgt gcaacgcctg cgggctctat cacaaaatga 1440
   acggacagaa ccggcccctc attaagccca agcgaaggct gtctgcagcc aggagagcag 1500
   ggacgtcctg tgcgaactgt cagaccacca caaccacact ctggaggagg aatgccaatg 1560
   gggaccctgt ctgcaatgcc tgtgggctct actacaagct tcacaatatt aacagacccc 1620
   tgactatgaa gaaggaaggc atccagacca gaaaccgaaa aatgtctagc aaatccaaaa 1680
   agtgcaaaaa agtgcatgac tcactggagg acttccccaa gaacagctcg tttaacccgg 1740
   ccgccctctc cagacacatg tcctccctga gccacatctc gcccttcagc caccccagcc 1800
   acatgctgac cacgcccacg ccgatgcacc cgccatccag cctgtccttt ggaccacacc 1860
   acccctccag catggtcacc gccatgggtt agagccctgc tgatgctcac agggccccca 1920
   gcgagagtcc ctgcagtccc tttcgacttg catttttgca ggagcagtat catgaagcct 1980
   aaacgcgatg gatatatgtt tttgaaggca gaaagcaaaa ttatgcttgc cactttgcaa 2040
   aggagctcac tgtggtgtct gtgttccaac cactgaatct ggaccccatc tgtgaataag 2100
   ccattctgac tcatatcccc tatttaacag ggtctctagt gctgtgaaaa aaaaaaatgc 2160
   tgaacattgc atataactta tattgtaaga aatactgtac aatgacttta ttgcatctgg 2220
   gtagctgtaa ggcatgaagg atgccaagaa gtttaaggaa tatgggagaa atagtgtgga 2280
   aattaagaag aaactaggtc tgatattcaa atggacaaac tgccagtttt gtttcctttc 2340
   actggccaca gttgtttgat gcattaaaag aaaataaaaa aaagaaaaag agaaaagaaa 2400
   aaaaaagaaa aaagttgtag gcgaatcatt tgttcaaagc tgttggcctc tgcaaaggaa 2460
   ataccagttc gggcaatcag tgttaccgtt caccagttgc cattgagggt ttcagagagc 2520
   ctttttctag gcctacatgc tttgtgaaca agtccctgta attgttgttt gtatgtataa 2580
   ttcaaagcac caaaataaga aaagatgtag atttatttca tcatattata cagaccgaac 2640
   tgttgtataa atttatttac tgctagtctt aagaactgct ttctttcgtt tgtttgtttc 2700
   aatattttcc ttctctctca attttcgg 2728

<210> 154
<211> 15
<212> DNA
<213> Artificial Sequence

<220>
<223> katalytic domain of 10-23 type DNAzyme


<220>
<221> misc_feature
<222> (1)..(15)
<223> katalytic domain of 10-23 type DNAzyme

<400> 154
ggctagctac aacga 15

<210>  155
<211>  33
<212>  DNA
<213>  Artificial Sequence

<220>
<223>  10-23 type DNAzyme in

<220>
<221>  misc_feature
<222>  (1)..(33)
<223>  10-23 type DNAzyme

<400>  155
nnnnnnnnng gctagctaca acgannnnnn nnn                                    33

## Beschreibung

Die Erfindung betrifft ein Verfahren und ein Kit zur Diagnose eines molekularen Phänotyps eines an einer mit chronischen Entzündungen einhergehenden Erkrankung leidenden Patienten sowie ein Arzneimittel zur Behandlung eines solchen Patienten.

Chronische Entzündungen stellen einen zunehmenden medizinischen Problemkreis von hoher sozioökonomischen Bedeutung dar. Hierzu zählen insbesondere folgende Erkrankungsgruppen: Autoimmunerkrankungen und Erkrankungen des rheumatischen Formenkreises (Manifestationen an u. a. Haut, Lunge, Niere, Gefäßsystem, Nervensystem, Bindegewebe, Bewegungsapparat, endokrinem System), Allergische Soforttypreaktionen und Asthma, Chronisch-obstruktive Lungenerkrankungen (COPD), Arteriosklerose, Psoriasis und Kontaktekzem und Chronische Abstoßungsreaktionen nach Organ- und Knochenmarkstransplantation. Viele dieser Erkrankungen zeigen in den letzten Dekaden eine ansteigende Prävalenz nicht nur in den Industrienationen, sondern zum Teil weltweit. So leiden in Europa, Nordamerika, Japan und Australien mittlerweile über 20% der Bevölkerung an allergischen Erkrankungen und Asthma. Chronisch-obstruktive Lungenerkrankungen sind zurzeit die fünfthäufigste Todesursache weltweit und werden nach Berechnungen der WHO im Jahre 2020 die dritthäufigste Todesursache darstellen. Arteriosklerose mit den Folgeerkrankungen Herzinfarkt, Schlaganfall und peripherer arterielle Verschlusskrankheit nehmen in der Morbiditäts- und Mortalitätsstatistik weltweit eine führende Position ein. Psoriasis und Kontaktekzem sind zusammen mit der Neurodermitis die häufigsten chronischen Entzündungserkrankungen an der Haut überhaupt.

Aufgrund von bis heute nur unzureichend verstandenen Wechselwirkungen zwischen Umweltfaktoren und einer genetischen Disposition kommt es zu nachhaltigen Fehlregulationen des Immunsystems. Hierbei lassen sich für diese unterschiedlichen Erkrankungen folgende gemeinsame Prinzipien feststellen:
(A) Es kommt zur Entwicklung einer überschießenden Immunantwort gegen normalerweise für den Menschen harmlose Antigene. Diese Antigene können Bestandteile der Umwelt sein (z. B. Allergene, wie Pollen, Tierhaare, Nahrungsmittel, Milben, chemische Substanzen wie Konservierungsstoffe, Farbstoffe, Reinigungsmittel). In diesen Fällen entwickelt sich bei den Patienten eine allergische Reaktion. Im Falle von z. B. Aktiv- und Passivrauchern kommt es zu chronisch-obstruktiven Lungenerkrankungen (COPD). Andererseits kann das Immunsystem aber auch gegen Komponenten des eigenen Organismus reagieren, diese als fremd erkennen und eine Entzündungsreaktion dagegen in Gang setzen. In diesen Fällen entwickelt sich eine Autoimmunerkrankung. In jedem Falle werden harmlose, nicht-toxische Antigene fälschlicherweise als fremd bzw. gefährlich erkannt und eine unangemessene Entzündungsreaktion in Gang gesetzt.
(B) Die Erkrankungen verlaufen in Phasen zu denen die Initiation, Progression, also Fortschreiten der Entzündungsreaktion, und die damit assoziierte Destruktion und der Umbau mit Verlust von Organ-Funktionalität (sogenanntes Remodeling) zählen.
(C) Die Erkrankungen zeigen Patienten-spezifische sub-phänotypische Ausprägungsmerkmale.
(D) An der Initiation, Aufrechterhaltung und den Destruktions- und Umbauprozessen sind Komponenten der angeborenen und erworbenen Immunität nachhaltig beteiligt. Unter dem Einfluss der angeborenen Immunität (wichtige Komponenten: Antigenpräsentierende Zellen mit ihren diversen Populationen und das Komplementsystem) kommt es zu Aktivierung und Differenzierung der Zellen des adaptiven Immunsystems (wichtige Komponenten: T-und B-Lymphozyten). Die T-Zellen übernehmen zentrale Funktionen im weiteren Verlauf indem sie in hoch-spezialisierte Effektoren differenzieren.

Hierbei aktivieren und erwerben sie bestimmte Effektormechanismen, zu denen insbesondere folgende Funktionen zählen: Antikörperproduktion, Kontrolle der Funktionalität von Effektorzellen des Immunsystems (wie z. B. neutrophile-, basophile-, eosinophile Granulozyten), Rückkopplung auf Funktionen des angeborenen Immunsystems, Beeinflussung der Funktionalität von nicht-hämatopoetischen Zellen wie z. B. Epithel, Endothel, Bindegewebe, Knochen und Knorpel und vor allem neuronale Zellen. Hier kommt es zu einer besonderen Wechselwirkung zwischen Immun- und Nervensystem, aus dem sich das Konzept der Neuro-immunologischen Interaktion bei chronischen Entzündungen entwickelt hat.

Da die bereits angesprochenen T-Zellen zentrale Funktionen im Krankheitsverlauf übernehmen ist ein Verständnis ihrer Spezialisierung entscheidend. Eine komplexe Signaltransduktionskaskade ist an der Differenzierung von naiven CD4⁺ Zellen zu Th1- bzw. Th2-Zellen beteiligt.

Die Stimulation über den T-Zellrezeptor durch den entsprechenden Peptid-MHC-Komplex induziert die klonale Expansion und programmierte Differenzierung von CD4+ T-Lymphozten zu T-Helfer (Th) 1- oder Th2-Zellen. Die Unterscheidung dieser beiden Subtypen erfolgt aufgrund ihrer Zytokin-Profile. Th1-Zellen produzieren Interferon-γ (INFγ), Interleukin 2 (IL-2) und Tumor-Nekrose-Faktor-α, wohingegen Th2-Zellen IL-4, IL-5, IL-9 und IL-13 sezernieren. Bakterielle und virale Infektionen induzieren eine Immunantwort, die von Th1-Zellen dominiert wird. Auf der anderen Seite regulieren Th2-Zellen die IgE Produktion gegen Parasiten. Dabei besteht zwischen Th1-und Th2-Zellen ein Gleichgewicht. Die Zerstörung dieses Gleichgewichtes verursacht Krankheiten, so ist eine überschießende Th1- Zellenantwort assoziiert mit Autoimmunerkrankungen, während allergischen Erkrankungen eine verstärkte Th2-Zellantwort zugrunde liegt.

Es ist bekannt, dass Th1-Zytokine in die Pathogenese von Autoimmunerkrankungen wie z. B. Autoimmunuveitis, experimentelle allergische Enzephalomyelitis, Typ 1 Diabetes mellitus oder Morbus Crohn involviert sind, während Th2-Zytokine (IL-4, IL-5, IL-13 bzw. IL-9) an der Entstehung von chronisch entzündlichen Atemwegserkrankungen, wie z. B. Atemwegseosinophilie, Asthma, Mukus-Hypersekretion und Atemwegs-Hyperreagibilität beteiligt sind. Grundlage dieser Erkrankungen sind pathophysiologische Veränderungen während der Produktion von charakteristischen Zytokinen durch antigenspezifische Th-Zellen. Th2-Zell-Subpopulationen in der Lunge und den Atemwegen rufen im Tiermodell die charakteristischen Symptome des Asthma bronchiale hervor.

An der Entstehung von Autoimmunerkrankungen und chronischer Entzündungsreaktionen sind u.a. zwei Transkriptionsfaktoren beteiligt: der Th1-Zell-spezifische Transkriptionsfaktor Tbet und der Th2-Zell-spezifische Transkriptionsfaktor GATA-3.

Der Th1-Zell-spezifische Transkriptionsfaktor Tbet ist vor allem für die Differenzierung von naiven CD4⁺ T-Zellen zu Th1-Zellen verantwortlich. Seine Expression wird über die Signaltransduktionswege des T-Zell Rezeptors (TZR) und über INFγ-Rezeptor/STAT1 kontrolliert. Tbet transaktiviert das endogene INFγ-Gen und induziert die INFγ Produktion. Die in vivo Funktion von Tbet wird in Knock-Out- Mäusen (Tbet-/-) bestätigt. In Tbet defizienten Mäuse ist die Menge an Th2-Zytokinen erhöht.

Bekannt ist die Funktion von Tbet in mucosalen T-Zellen bei der Entstehung von entzündlichen Darmerkrankungen. Der Transkriptionsfaktor Tbet induziert spezifisch die Entwicklung von Th1-Zellen und kontrolliert die INFγ-Produktion in diesen Zellen. Durch die Inhibition von Tbet wird die Balance zwischen Th1-und Th2-Zellen zugunsten von Th2-Zellen verschoben.

Viele entzündliche Erkrankungen hingegen, wie beispielsweise das allergische Asthma, sind assoziiert mit einer Aktivierung von Th2-Zellen. Th2-Zellen spielen eine wesentliche Funktion bei der Entwicklung allergischer Erkrankungen, insbesondere verschiedener Asthmaerkrankungen. Die dazu erforderliche Differenzierung von Th0-Zellen zu Th2-Zellen ist abhängig von dem Transkriptionsfaktor GATA-3. GATA-3 ist ein Mitglied der GATA-Familie der Transkriptionsfaktoren.

Der Th2-Zell-spezifische Transkriptionsfaktor GATA-3 ist vor allem für die Differenzierung von naiven CD4⁺ T-Zellen zu Th2-Zellen verantwortlich. Die Th2-Zelldifferenzierung wird dabei hauptsächlich durch zwei Signalübertragungswege, den T-Zell Rezeptor- (TZR) und den IL-4 Rezeptor-Weg, gesteuert: Vom TZR weitergeleitete Signale aktivieren sowohl die Th2 zellspezifischen Transkriptionsfaktoren c-Maf und GATA-3 als auch die Transkriptionsfaktoren NFAT und AP-1. Die Aktivierung des IL-4 Rezeptors führt zur Bindung von STAT6 an die zytoplasmatische Domäne des IL-4 Rezeptors, wo er von Jak1- und Jak3-Kinasen phosphoryliert wird. Die Phosphorylierung ihrerseits führt zur Dimerisierung und Translokation von STAT6 in den Nukleus, wo STAT6 die Transkription von GATA-3 und anderen Genen aktiviert. GATA-3 ist ein Zinkfinger-Transkriptionsfaktor, der ausschließlich in reifen Th2-Zellen exprimiert wird, nicht in Th1-Zellen.

Th2-Zellen produzieren Zytokine wie beispielsweise IL-4, IL-5, IL-6, IL-13 und GM-CSF. Die Polarisierung zu Th2 inhibiert eine Th1-Differenzierung durch Suppression von Tbet und umgekehrt. Die Expression von GATA-3 ist jedoch nicht auf T-Zellen beschränkt. Eine Expression von GATA-3 konnte auch in eosinophilen und basophilen Granulocyten, Mastzellen und Epithelzellen nachgewiesen werden. GATA-3 spielt eine zentrale Rolle in der Immunpathogenese von chronischen Entzündungserkrankungen, insbesondere von allergischem Asthma.

Etablierte Präparate zur Behandlung von chronischen Entzündungserkrankungen sind u.a. Kortikosteroide, Anti-Leukotriene, Immunsuppressiva und Anti-IgE monoklonale Antikörper. Asthmapatienten sprechen jedoch unterschiedlich gut auf diese Therapeutika an. Lange Zeit blieb ungeklärt, worauf diese Unterschiede in der Wirksamkeit zurückzuführen sind. In Konsequenz musste am Patienten die passende Therapie im Einzelfall mehr oder weniger nach dem Prinzip "Versuch und Irrtum" eingestellt werden.

Erst kürzlich konnte jedoch festgestellt werden, dass sich beispielsweise an Asthma leidende Patienten in Untergruppen weiter aufteilen lassen (Woodruff et al., 2009, T-helper Type 2-driven Inflammation Defines Major Subphenotypes of Asthma, Am J Respir Crit Care Med, Vol 180, 388-395). So wurde gezeigt, dass es unter Asthmapatienten wenigstens zwei Untergruppen gibt, die mit "Th2 high" und "Th2 low" bezeichnet wurden. Die Untergruppe "Th2 high" der Asthmapatienten besitzt dabei u.a. eine erhöhte Expression des Gens POSTN, welches für das Protein Periostin kodiert, sowie der Gene für IL-13 und IL-5. Die Gruppe "Th2 low" der getesteten Asthmapatienten zeigt eine niedrige POSTN-Genexpression, vergleichbar zu einer Kontrollgruppe gesunder Personen. Diese unterschiedlichen molekularen Phänotypen könnten eine Ursache für die unterschiedliche Wirksamkeit gängiger Therapeutika sein. So konnte für die Untergruppe "Th2 high" ein verbessertes Ansprechverhalten auf eine Behandlung mit Kortikosteroiden festgestellt werden.

Es konnte weiterhin gefunden werden, dass zwei Gruppen von Asthmapatienten, nämlich "Th2 high" und "Th2 low", unterschiedlich gut auf eine Therapie mit einem humanisierten monoklonalen Antikörper gegen IL-13 ansprechen (Corren et al., 2011, Lebrikizumab Treatment in Adults with Asthma, The new england journal of medicine, 10.1056/NEJMoa1106469). Dabei erfolgte zunächst eine empirische Eingruppierung von Asthmapatienten in die Gruppe "Th2-high", wenn die Werte für Serum-IgE höher als 100 IU/ml und die Zahl der eosinophilen Granulocyten bei 0,14×10⁹ Zellen pro Liter oder höher waren. Bei entsprechenden Werten jeweils darunter erfolgte eine Eingruppierung in die Gruppe "Th2-low". Alternativ erfolgte eine Klassifizierung nach dem Serum-Periostin-Spiegel, welcher als Ersatzmarker (Surrogatmarker) für das in Blut- oder Atemwegsproben schwierig nachweisbare Th2-Zytokin IL-13 dient. Dabei wird die Tatsache ausgenutzt, dass IL-13 u.a. die Expression des Periostin kodierenden Gens POSTN in Epithelzellen in vitro induziert (Woodruff et al., 2007, Proc Natl Acad Sci USA., 104(40):15858-63. Genome-wide profiling identifies epithelial cell genes associated with asthma and with treatment response to corticosteroids.). Patienten mit einem Serum-Periostin-Spiegel über dem Durchschnitt wurden nach Corren et al., 2011, in eine Gruppe "Periostin-high" eingeordnet. Für die genannten Untergruppen "Th2-high" und "Periostin-high" konnte tendenziell ein besseres Ansprechverhalten auf eine Behandlung mit Anti-IL-13-Antikörper beschrieben werden.

Nach der WO 2009/124090 A1 wurde ebenfalls eine gewisse Klassifikation von Asthmapatienten vorgeschlagen, wobei die Genexpression einer Vielzahl von Kandidatengenen, wie beispielsweise POSTN, CLCA1 und SERPINB2, herangezogen wurde. Da diese Gene bekanntermaßen durch die Th2-Zytokine IL-4 oder IL-13 hochreguliert werden, wurde das Cluster auch als "IL-4/IL-13-Signatur" bezeichnet. Neben der Messung des Serum-Periostin-Spiegels sowie der entsprechenden mRNA Menge wurde dabei eine Bestimmung der Werte für Serum-lgE und der Zahl der eosinophilen Granulocyten beschrieben.

Ein Nachteil der Patientenstratifizierung auf Grundlage dieser Genexpression, vor allem von POSTN, ist, dass neben einer "IL-4/IL-13-Signatur" auch das Zytokin IL-5 eine bedeutende Rolle in der Genese von Asthma spielt. Darüber hinaus ist die Rolle des Proteins Periostin in der Immunkaskade und damit der Krankheitsentstehung unbekannt.

Es stellte sich somit die Aufgabe, einen Biomarker aufzufinden, der zur zuverlässigen und gleichzeitig klinisch praktikablen molekularen Phänotypisierung von einem an einer Erkrankung, die mit chronischen Entzündungen einhergeht, leidenden humanen Patienten in die Gruppen "Th2-high" oder "Th2-low" bzw. "Th1-high" oder "Th1-low" geeignet ist. Weiterhin sollte der auf diese Weise klassifizierte Patient mit einem spezifisch für diese Untergruppe besonders wirksamen Therapeutikum behandelbar sein. Der Biomarker soll dazu eine individuelle Vorhersage über die Wirksamkeit eines Therapeutikums in Bezug auf einen Patienten, insbesondere einen Asthmapatienten, ermöglichen.

Die genannte Aufgabe wird erfindungsgemäß durch ein Verfahren zur Diagnose eines molekularen Phänotyps eines an einer Erkrankung, die mit chronischen Entzündungen einhergeht, leidenden humanen Patienten gelöst, wobei der molekulare Phänotyp ausgewählt ist aus der Gruppe bestehend aus den Untergruppen "Th2 high", "Th2 low", "Th1 high" und "Th1 low" und die Genexpression von GATA-3 und/oder Tbet in einem biologischen Isolat des Patienten gemessen und für die Zuordnung zu einem molekularen Phänotyp der Erkrankung genutzt wird. Die nähere Klassifizierung eines an einer Erkrankung, die mit chronischen Entzündungen einhergeht, leidenden humanen Patienten erfolgt dabei durch Messung der Genexpression des Transkriptionsfaktors GATA-3 und/oder des Transkriptionsfaktors Tbet. Wie oben bereits einleitend ausgeführt, sind an der Entstehung von Autoimmunerkrankungen und chronischen Entzündungsreaktionen der Th1-Zell-spezifische Transkriptionsfaktor Tbet und der Th2-Zell-spezifische Transkriptionsfaktor GATA-3 beteiligt. Die Polarisierung zu Th2 inhibiert eine Th1-Differenzierung durch Suppression von Tbet und umgekehrt. Je nach Expressionsniveau von GATA-3 und/oder Tbet kann eine Zuordnung zu einem molekularen Phänotyp, also zu einer Untergruppe der mit chronischen Entzündungen einhergehenden Erkrankung, erfolgen. Mit dem erfindungsgemäßen Diagnoseverfahren kann die genannte molekulare Phänotypisierung ohne Schwierigkeiten im klinischen Alltag mit hoher Aussagekraft vorgenommen werden.

Die Transkriptionsfaktoren GATA-3 und Tbet sind die zentralen Schlüsselmoleküle bei der Entstehung von Th1- bzw. Th2-abhängigen chronischen Entzündungserkrankungen. Deswegen stellt die direkte Messung der Protein- bzw. mRNA-Expression einen bestmöglichen Patientenstratifizierungsansatz dar, da keine zwischengeschalteten Mechanismen das Ergebnis möglicherweise verfälschen können.

Nach einer bevorzugten Ausführungsform des Verfahrens wird das Expressionsniveau von GATA-3 und/oder Tbet über die Protein- oder die mRNA-Menge bestimmt. Dabei kann die Protein-Menge mit Hilfe eines Immunassays quantitativ bestimmt werden. Der Immunassay ist vorzugsweise ein Enzyme-linked Immunosorbent Assay (ELISA)-Test, ein Radioimmunassay (RIA), ein Elektrochemilumineszenz (ECL)-Immunassay, ein CLIA (chemoluminescence-linked Immunosorbent assay), ein FLIA (fluorescence-linked Immunosorbent assay) oder ein Multiplex-Assay.

Die genannten Assays bieten, neben hoher Sensibilität und Spezifität, alle den Vorteil einer möglichen Automatisierung und sind somit für den klinischen Alltag besonders gut geeignet. Selbstverständlich kann im Sinne der vorliegenden Erfindung gegebenenfalls jeder andere geeignete Test zur quantitativen Erfassung der Protein-Menge von GATA-3 und/oder Tbet auswählt werden. Weiterhin kann das Expressionsniveau von GATA-3 und/oder Tbet über massenspektrometrische Verfahren, chromatographische Verfahren, wie Gaschromatographie, flüssigkeitsbasierende Verfahren mit Festphasentrennung, wie HPLC, oder mikro- und nanofluidische Verfahren erfolgen.

Das Verfahren kann gegebenenfalls zur Ermittlung der Expression von GATA-3 oder Tbet mit Hilfe eines ELISA-Tests folgende Schritte umfassen:
- Herstellung eines Lysats durch Zellaufschluss;
- Zugabe des Lysats in eine Vertiefung einer Mikrotiterplatte, die mit einem ersten GATA-3- oder Tbet-spezifischen Antikörper beschichtet ist;
- Waschen der Mikrotiterplatte;
- Zugabe eines zweiten GATA-3- oder Tbet-spezifischen Antikörpers in die Vertiefung der Mikrotiterplatte;
- Waschen der Mikrotiterplatte;
- Detektion und Quantifizierung des GATA-3 oder Tbet Proteins.

Zum Zweck der Detektion kann der zweite spezifische Antikörper beispielsweise mit Biotin markiert sein und eine separate Zugabe eines an Streptavidin gekoppelten Enzyms erfolgen. Der zweite spezifische Antikörper kann aber auch direkt mit einem Enzym gekoppelt sein. Wenn es zweckmäßig ist, kann auch ein gegen den zweiten spezifischen Antikörper gerichteter dritter Antikörper, der mit einem Enzym gekoppelt ist, verwendet werden.

Das Enzym ist vorzugsweise eine Peroxidase oder alkalische Phosphatase und wird mit einem geeigneten Substrat umgesetzt, das zur Colorimetrie oder Chemilumineszenz und ähnlichem geeignet ist.

Die mRNA-Menge von GATA-3 und/oder Tbet kann nach einem weiteren Aspekt der vorliegenden Erfindung zusätzlich oder in Alternative zur genannten Erfassung der Protein-Menge bestimmt werden. Dazu ist bevorzugt eine PCR, besonders bevorzugt eine qPCR, oder ein Micro-Array-Chip geeignet. Dem Fachmann ist bekannt, wie GATA-3 und Tbet spezifische Sonden bzw. Primer für die genannten Nachweismethoden auszuwählen sind.

Das biologische Isolat wurde gemäß einer bevorzugten Ausführung des Verfahrens aus Vollblut, Urin, Sputum, einer broncho-alveolären Lavage (BAL), einer Biopsie, einem Bürstenabstrich, Liquor, Trachealsekret, Seminalflüssigkeit, Ascitesflüssigkeit, Speichel, Punkttat oder Lymphflüssigkeit gewonnen. Der Fachmann kennt die Routinemethoden zur Gewinnung von geeignetem biologischem Isolat.

GATA-3 und Tbet sind Proteine, die als Transkriptionsfaktoren ihre Wirkung im Zellkern von T-Helferzellen des Subtyps Th1 und Th2 entfalten. Um die Konzentration dieser beiden nukleären Proteine in einem bestimmten Volumen eines biologischen Isolats, wie in einem bestimmten Volumen an Vollblut oder ähnlichem, zu bestimmen, müssen Zellen, welche GATA-3 und Tbet bilden, zunächst isoliert und danach lysiert werden. Ein direkter Nachweis dieser Proteine aus menschlichem Serum oder Plasma ist kaum möglich, da sie dort nicht in detektierbarer Konzentration vorhanden sind. Eine Analyse von GATA-3 und Tbet erfolgt daher gegebenenfalls in vier Stufen:
- Abtrennung und Isolierung der GATA-3/Tbet exprimierenden Zellen von den übrigen zellulären Bestandteilen des Vollblutes
- Aufschluss der Zellen und Freisetzung der intrazellulären/nukleären Proteine
- Messung der Konzentration von GATA-3 und Tbet und
- Normierung der gefundenen Konzentrationen von GATA-3 und Tbet.

Gemäß einer vorteilhaften Weiterentwicklung umfasst das erfindungsgemäße Verfahren, unabhängig davon, ob das Expressionsniveau von GATA-3 und/oder Tbet über die Protein- oder die mRNA-Menge bestimmt wird, auch einen oder mehrere der folgenden Schritte:
(i) Isolierung von Leukocyten, vorzugsweise mittels Ficoll-Gradientenzentrifugation;
(ii) Anreicherung von Leukocyten, vorzugsweise mittels Größenausschluss-Filtration; oder
(iii) Anreicherung von Th1/Th2 Zellen, insbesondere CD4⁺-T-Zellen mit Hilfe zellspezifischer Antikörper, welche vorzugsweise an magnetische Beads gekoppelt sind.

Die genannten Schritte (i) - (iii) werden vor dem Zellaufschluss durchgeführt und ermöglichen jeweils eine Erhöhung der Empfindlichkeit sowie der Aussagekraft des Diagnoseverfahrens, da insbesondere in den Leukocyten die Gene GATA-3 und Tbet differenziell exprimiert werden.

Nach einem weiteren Aspekt der vorliegenden Erfindung erfolgt eine Zuordnung des Patienten zu einem molekularen Phänotyp der Untergruppe "Th2 high", wenn mindestens eine der folgenden Bedingungen erfüllt ist:
a) die GATA-3 Genexpression im biologischen Isolat ist höher als ein definierter Referenzwert,
b) das Verhältnis von GATA-3 : Tbet Genexpression im biologischen Isolat ist höher als ein definierter Referenzwert.

Die Untergruppe "Th2 high" ist also entweder durch eine hohe absolute GATA-3 Genexpression gegenüber einem definierten Referenzwert gekennzeichnet. Dabei kann als Referenzwert - im Falle der Messung der Proteinmenge - ein entsprechender Wert des GATA-3 Proteingehalts im Isolat einer gesunden Person herangezogen werden. Es können aber auch absolute Referenzwerte herangezogen werden. Im Falle der Messung der mRNA-Menge kann als Referenzwert ein entsprechender Wert der GATA-3 mRNA-Menge im Isolat einer gesunden Person herangezogen werden. Es können aber auch wiederum absolute Referenzwerte, wie beispielsweise Kopien/ml, herangezogen werden.

Eine Zuordnung des Patienten zu dem genannten molekularen Phänotyp der Untergruppe "Th2 high" erfolgt nach einer vorteilhaften Ausführungsform des erfindungsgemäßen Verfahrens, wenn alternativ oder zusätzlich zur erhöhten GATA-3 Genexpression das Verhältnis von GATA-3 : Tbet Genexpression im biologischen Isolat höher als ein definierter Referenzwert ist. Dabei kann als Referenzwert ein entsprechender Wert des Verhältnisses von GATA-3 : Tbet Genexpression im Isolat einer gesunden Person herangezogen werden. Die Bestimmung des Verhältnisses von GATA-3 : Tbet Genexpression erhöht die Sicherheit der Aussage, da dabei neben der GATA-3 Genexpression als zusätzlicher Parameter die Tbet Genexpression festgestellt wird. Da die beiden Transkriptionsfaktoren sich in ihrer Expression, wie einleitend beschrieben, gegenläufig regulieren, stellt die Einbeziehung von Tbet eine interne Kontrolle für die Messung der GATA-3 Genexpression dar.

Nach einer vorteilhaften Weiterentwicklung umfasst das erfindungsgemäße Verfahren zur Zuordnung des Patienten zu dem molekularen Phänotyp der Untergruppe "Th2 high" die Schritte:
- Freisetzung von Proteinen oder RNA aus Zellen eines biologischen Isolats des Patienten;
- Ermittlung des Expressionsniveaus der Proteine oder der mRNA für GATA-3 und/oder Tbet;
- Einordnung des Patienten in eine die Untergruppe "Th2 high", wenn mindestens eine der vorstehend unter a) oder b) genannten Bedingungen zutrifft.

Alternativ findet zusätzlich zur genannten Bestimmung der GATA-3 und/oder Tbet Genexpression noch eine Bestimmung weiterer Parameter zur sicheren Einordnung in die Untergruppe "Th2 high" statt. So kann beispielsweise der Serum-IgE-Spiegel und die Zahl der eosinophilen Granulocyten gemessen werden. Eine Zuordnung zur Untergruppe "Th2 high" erfolgt dann, wenn weiterhin der Serum-lgE-Spiegel höher als 100 IU/ml und/oder die Zahl der eosinophilen Granulocyten bei 0,14×10⁹ Zellen pro Liter oder höher liegt. Alternativ kann bei Bedarf auch noch die Konzentration an Stickstoffmonoxid in der ausgeatmeten Luft, also eine Bestimmung des FeNO-Werts, durchgeführt werden.

Eine weitere vorteilhafte Ausführungsform des erfindungsgemäßen Verfahrens betrifft eine Zuordnung des Patienten zu einem molekularen Phänotyp der Untergruppe "Th2 low", wenn mindestens eine der folgenden Bedingungen erfüllt ist:
a) die GATA-3 Genexpression im biologischen Isolat ist niedriger als ein definierter Referenzwert,
b) das Verhältnis von GATA-3 : Tbet Genexpression im biologischen Isolat ist niedriger als ein definierter Referenzwert.

Die Untergruppe "Th2 low" ist also entweder durch eine niedrige absolute GATA-3 Genexpression gegenüber einem definierten Referenzwert gekennzeichnet. Dabei kann als Referenzwert - im Falle der Messung der Proteinmenge - ein entsprechender Wert des GATA-3 Proteingehalts im Isolat einer gesunden Person herangezogen werden. Hier ist zu beachten, dass bei einem Patienten der Untergruppe "Th2 low" mit einer mit chronischen Entzündungen einhergehenden Erkrankung die absolute GATA-3 Genexpression regelmäßig dennoch höher sein wird als in einem Isolat eines gesunden Patienten. Die absolute GATA-3 Genexpression kann jedoch auch niedriger als bei einem Gesunden sein. Jedenfalls ist die GATA-3 Genexpression aber nicht so hoch wie für die Untergruppe "Th2 high" beschrieben. Eine Zuordnung des Patienten zur Untergruppe "Th2 low" erfolgt somit, wenn dessen GATA-3 Proteingehalt gegenüber einem Isolat einer gesunden Person, wenn überhaupt, nur moderat erhöht ist. Es können aber auch feste Referenzwerte herangezogen werden. Im Falle der Messung der mRNA-Menge kann als Referenzwert ein entsprechender Wert der GATA-3 mRNA-Menge im Isolat einer gesunden Person herangezogen werden, wobei eine Zuordnung des Patienten zur Untergruppe "Th2 low" erfolgt, wenn dessen GATA-3 mRNA-Menge geringer als in einer entsprechenden Probe eines Gesunden oder jedenfalls nicht wesentlich erhöht ist. Es können aber auch wiederum feste Referenzwerte herangezogen werden.

Eine Zuordnung des Patienten zu dem genannten molekularen Phänotyp der Untergruppe "Th2 low" erfolgt nach einer vorteilhaften Ausführungsform des erfindungsgemäßen Verfahrens, wenn alternativ oder zusätzlich zur verminderten GATA-3 Genexpression das Verhältnis von GATA-3 : Tbet Genexpression im biologischen Isolat niedriger als ein definierter Referenzwert ist. Dabei kann als Referenzwert ein entsprechender Wert des Verhältnisses von GATA-3 : Tbet Genexpression im Isolat einer gesunden Person herangezogen werden. Die Bestimmung des Verhältnisses von GATA-3 : Tbet Genexpression erhöht auch hier die Sicherheit der Aussage, da dabei neben der GATA-3 Genexpression als zusätzlicher Parameter die Tbet Genexpression festgestellt wird. Da die beiden Transkriptionsfaktoren sich in ihrer Expression, wie einleitend beschrieben, gegenläufig regulieren, stellt die Einbeziehung von Tbet in gewisser Hinsicht eine interne Kontrolle für die Messung der GATA-3 Genexpression dar.

Nach einer vorteilhaften Weiterentwicklung umfasst das erfindungsgemäße Verfahren zur Zuordnung des Patienten zu dem molekularen Phänotyp der Untergruppe "Th2 low" die Schritte:
- Freisetzung von Proteinen oder RNA aus Zellen eines biologischen Isolats des Patienten;
- Ermittlung des Expressionsniveaus der Proteine oder der mRNA für GATA-3 und/oder Tbet;
- Einordnung des Patienten in eine die Untergruppe "Th2 low", wenn mindestens eine der vorstehend unter a) oder b) genannten Bedingungen zutrifft.

Alternativ können zusätzlich zur genannten Bestimmung der GATA-3 und/oder Tbet Genexpression wiederum noch weitere Parameter zur sicheren Einordnung in die Untergruppe "Th2 low" bestimmt werden. So kann beispielsweise der Serum-IgE-Spiegel und die Zahl der eosinophilen Granulocyten gemessen werden. Eine Zuordnung zur Untergruppe "Th2 low" erfolgt dann, wenn weiterhin der Serum-lgE-Spiegel niedriger als 100 IU/ml und/oder die Zahl der eosinophilen Granulocyten unter 0,14×10⁹ Zellen pro Liter liegt. Alternativ kann bei Bedarf auch noch die Konzentration an Stickstoffmonoxid in der ausgeatmeten Luft, also eine Bestimmung des FeNO-Werts, durchgeführt werden.

Gemäß einer weiteren erfindungsgemäßen Ausführungsform erfolgt eine Zuordnung des Patienten zu einem molekularen Phänotyp der Untergruppe "Th1 high", wenn mindestens eine der folgenden Bedingungen erfüllt ist:
a) die Tbet Genexpression im biologischen Isolat ist höher als ein definierter Referenzwert,
b) das Verhältnis von Tbet : GATA-3 Genexpression im biologischen Isolat ist höher als ein definierter Referenzwert.

Die Untergruppe "Th1 high" ist also entweder durch eine hohe absolute Tbet Genexpression gegenüber einem definierten Referenzwert gekennzeichnet. Dabei kann als Referenzwert - im Falle der Messung der Proteinmenge - ein entsprechender Wert des Tbet Proteingehalts im Isolat einer gesunden Person herangezogen werden, wobei eine Zuordnung des Patienten zur Untergruppe "Th1 high" erfolgt, wenn dessen Tbet Proteingehalt erhöht ist. Es können aber auch feste Referenzwerte herangezogen werden. Im Falle der Messung der mRNA-Menge kann als Referenzwert ein entsprechender Wert der Tbet mRNA-Menge im Isolat einer gesunden Person herangezogen werden, wobei eine Zuordnung des Patienten zur Untergruppe "Th1 high" erfolgt, wenn dessen Tbet mRNA-Menge erhöht ist. Es können aber auch wiederum feste Referenzwerte herangezogen werden.

Eine Zuordnung des Patienten zu dem genannten molekularen Phänotyp der Untergruppe "Th1 high" erfolgt nach einer vorteilhaften Ausführungsform des erfindungsgemäßen Verfahrens, wenn alternativ oder zusätzlich zur erhöhten Tbet Genexpression das Verhältnis von Tbet : GATA-3 Genexpression im biologischen Isolat höher als ein definierter Referenzwert ist. Dabei kann als Referenzwert ein entsprechender Wert des Verhältnisses von Tbet : GATA-3 Genexpression im Isolat einer gesunden Person herangezogen werden. Die Bestimmung des Verhältnisses von Tbet : GATA-3 Genexpression erhöht auch hier die Sicherheit der Aussage, da dabei neben der Tbet Genexpression als zusätzlicher Parameter die GATA-3 Genexpression festgestellt wird und die Einbeziehung von GATA-3 eine interne Kontrolle für die Messung der Tbet Genexpression darstellt.

Nach einer vorteilhaften Weiterentwicklung umfasst das erfindungsgemäße Verfahren zur Zuordnung des Patienten zu dem molekularen Phänotyp der Untergruppe "Th1 high" die Schritte:
- Freisetzung von Proteinen oder RNA aus Zellen eines biologischen Isolats des Patienten;
- Ermittlung des Expressionsniveaus der Proteine oder der mRNA für Tbet und/oder GATA-3;
- Einordnung des Patienten in eine die Untergruppe "Th1 high", wenn mindestens eine der vorstehend unter a) oder b) genannten Bedingungen zutrifft.

Ein weiterer Aspekt der vorliegenden Erfindung betrifft ein Verfahren, das eine Zuordnung des Patienten zu einem molekularen Phänotyp einer Untergruppe "Th1 low" ermöglicht, wenn mindestens eine der folgenden Bedingungen erfüllt ist:
a) die Tbet Genexpression im biologischen Isolat ist niedriger als ein definierter Referenzwert,
b) das Verhältnis von Tbet : GATA-3 Genexpression im biologischen Isolat ist niedriger als ein definierter Referenzwert.

Die Untergruppe "Th1 low" ist also entweder durch eine niedrige absolute Tbet Genexpression gegenüber einem definierten Referenzwert gekennzeichnet. Dabei kann als Referenzwert - im Falle der Messung der Proteinmenge - ein entsprechender Wert des Tbet Proteingehalts im Isolat einer gesunden Person herangezogen werden. Hier ist zu beachten, dass bei einem Patienten der Untergruppe "Th1 low" mit einer mit chronischen Entzündungen einhergehenden Erkrankung die absolute Tbet Genexpression dennoch höher sein kann als in einem Isolat eines gesunden Patienten. Die absolute Tbet Genexpression kann jedoch auch niedriger als bei einem Gesunden sein. Jedenfalls ist die Tbet Genexpression aber nicht so hoch wie für die Untergruppe "Th1 high" beschrieben. Eine Zuordnung des Patienten zur Untergruppe "Th1 low" erfolgt somit, wenn dessen Tbet Proteingehalt gegenüber einem Isolat einer gesunden Person, wenn überhaupt, jedoch nicht wesentlich erhöht ist. Es können aber auch feste Referenzwerte herangezogen werden. Im Falle der Messung der mRNA-Menge kann als Referenzwert ein entsprechender Wert der Tbet mRNA-Menge im Isolat einer gesunden Person herangezogen werden, wobei eine Zuordnung des Patienten zur Untergruppe "Th1 low" erfolgt, wenn dessen Tbet mRNA-Menge geringer als in einer entsprechenden Probe eines Gesunden oder jedenfalls nicht wesentlich erhöht ist. Es können aber auch wiederum feste Referenzwerte herangezogen werden.

Eine Zuordnung des Patienten zu dem genannten molekularen Phänotyp der Untergruppe "Th1 low" erfolgt nach einer vorteilhaften Ausführungsform des erfindungsgemäßen Verfahrens, wenn alternativ oder zusätzlich zur verminderten Tbet Genexpression das Verhältnis von Tbet : GATA-3 Genexpression im biologischen Isolat niedriger als ein definierter Referenzwert ist. Dabei kann als Referenzwert ein entsprechender Wert des Verhältnisses von Tbet : GATA-3 Genexpression im Isolat einer gesunden Person herangezogen werden. Die Bestimmung des Verhältnisses von Tbet : GATA-3 Genexpression erhöht auch hier die Sicherheit der Aussage, da dabei neben der Tbet Genexpression als zusätzlicher Parameter die GATA-3 Genexpression festgestellt wird und die Einbeziehung von GATA-3 in gewisser Hinsicht eine interne Kontrolle für die Messung der Tbet Genexpression darstellt.

Nach einer vorteilhaften Weiterentwicklung umfasst das erfindungsgemäße Verfahren zur Zuordnung des Patienten zu dem molekularen Phänotyp der Untergruppe "Th1 low" die Schritte:
- Freisetzung von Proteinen oder RNA aus Zellen eines biologischen Isolats des Patienten;
- Ermittlung des Expressionsniveaus der Proteine oder der mRNA für Tbet und/oder GATA-3;
- Einordnung des Patienten in eine die Untergruppe "Th1 low", wenn mindestens eine der vorstehend unter a) oder b) genannten Bedingungen zutrifft.

Alternativ können zusätzlich zur genannten Bestimmung der GATA-3 und/oder Tbet Genexpression noch weitere Parameter zur sicheren Einordnung in die Untergruppe "Th1 high" und "Th1 low" bestimmt werden. So kann beispielsweise die Zahl der eosinophilen Granulocyten oder der Serum lgE-Spiegel gemessen werden. Eine Zuordnung zur Untergruppe "Th1 high" erfolgt dann, wenn der Serum-IgE-Spiegel niedriger als 100 IU/ml und/oder die Zahl der eosinophilen Granulocyten unter 0,14x10⁹ Zellen pro Liter liegt. Anderenfalls erfolgt eine Zuordnung zu der Untergruppe "Th1 low". Alternativ kann bei Bedarf auch noch die Konzentration an Stickstoffmonoxid in der ausgeatmeten Luft, also eine Bestimmung des FeNO-Werts, durchgeführt werden.

Um Unterschiede in der Probenvorbereitung zu berücksichtigen, kann eine Normierung der Konzentrationen von GATA-3 und Tbet durchgeführt werden. Unterschiede in der Probenvorbereitung können z.B. entstehen durch unterschiedliche Zellzahlen, die lysiert werden, durch unterschiedliche Lyseeffizienzen der einzelnen Proben oder durch unterschiedlichen Gehalt an den verschiedenen Zelltypen innerhalb der Zellpräparationen. Möglichkeiten der Normierung sind erfindungsgemäß unter anderem: Normierung auf den Gesamt-Proteingehalt des Zelllysates, Normierung auf die Zellzahl, die lysiert wurde oder Normierung auf die Konzentration spezifischer Markerproteine, die spezifisch in bestimmten Zelltypen gefunden werden.

Die Patienten mit diagnostiziertem molekularen Phänotyp der Untergruppe "Th2 high" können unter Umständen gleichzeitig in die Untergruppe "Th1 low" eingeordnet werden. Auch können Patienten mit diagnostiziertem molekularen Phänotyp der Untergruppe "Th1 high" unter Umständen gleichzeitig in die Untergruppe "Th2 low" eingeordnet werden. Dies ist auf die oben dargestellte Tatsache zurückzuführen, dass die Polarisierung zu Th2 eine Th1-Differenzierung durch Suppression von Tbet inhibiert und umgekehrt.

Im Sinne der vorliegenden Erfindung werden Erkrankungen diagnostiziert bzw. therapiert, die mit chronischen Entzündungen einhergehen, wie z.B. Autoimmunerkrankungen, Erkrankungen des rheumatischen Formenkreises (Manifestationen an u. a. Haut, Lunge, Niere, Gefäßsystem, Nervensystem, Bindegewebe, Bewegungsapparat, Endokrinem System), Allergische Soforttypreaktionen und Asthma, Chronisch-obstruktive Lungenerkrankungen (COPD), Arteriosklerose, Psoriasis und Kontaktekzem sowie chronische Abstoßungsreaktionen nach Organ- und Knochenmarkstransplantation. Auch Tumorerkrankungen können erfindungsgemäß diagnostiziert bzw. therapiert werden, soweit GATA-3 oder Tbet an der Entstehung beteiligt und/oder als Folgeerscheinung dereguliert sind.

Im Sinne der vorliegenden Erfindung ist die chronische Entzündungserkrankung entweder Th2-bedingt, wie beispielsweise allergisches Asthma Bronchiale, Rhinokonjunktivitis, allergische Sinusitis, atopische Dermatitis, Nahrungsmittelallergien, Pemphigus, Ulcerative Colitis, parasitäre Erkrankungen, oder Th1-bedingt, wie beispielsweise Psoriasis, allergisches Kontaktekzem, Morbus Crohn, COPD, Rheumatoide Arthritis, Autoimmunerkrankungen, Typ 1 Diabetes mellitus oder MS.

Die oben genannte Aufgabe wird weiterhin erfindungsgemäß durch ein Arzneimittel zur Behandlung von Erkrankungen, die mit chronischen Entzündungen einhergehen, eines humanen Patienten mit einem molekularen Phänotyp gelöst, wobei der molekulare Phänotyp gemäß einem Verfahren nach einer oder mehreren Ausführungsformen des genannten erfindungsgemäßen Diagnoseverfahrens ermittelt wurde. Der identifizierte molekulare Phänotyp umfasst dabei insbesondere die Gruppen "Th1 low", "Th1 high", "Th2 low" oder "Th2 high".

Nach einer bevorzugten Ausführungsform enthält das genannte Arzneimittel eine für GATA-3 oder Tbet spezifische Ribonukleinsäure oder Desoxyribonukleinsäure, insbesondere ein für GATA-3 oder Tbet spezifisches DNAzym.

Ein generelles DNAzym-Modell stellt das "10-23"-Modell dar (Sontoro et al., 1997). DNAzyme des 10-23-Modells - auch als "10-23 DNAzyme" bezeichnet - besitzen eine katalytische Domäne von 15 Nukleotiden, welche von zwei Substratbindungsdomänen flankiert wird. Die katalytische Domäne besitzt dabei vorzugsweise die Sequenz ggctagctacaacga (SEQ ID No. 154). Die Länge der Substratbindungsdomänen ist variabel, sie sind entweder gleich oder unterschiedlich lang. In einer bevorzugten Ausführung, beträgt die Länge der Substratbindungsdomänen zwischen 6 und 14 Nukleotide, ganz besonders bevorzugt jeweils mindestens neun Nukleotide. Solche DNAzyme umfassen die allgemeine Sequenz nnnnnnnnnggctagctacaacgannnnnnnnn (SEQ ID NO 155). Besonders bevorzugt sind dabei Substratbindungsdomänen, die mRNA, kodierend für die Proteine GATA-3 und Tbet, binden.

Die angegebene katalytische zentrale Domäne ggctagctacaacga ist lediglich eine bevorzugte Ausführungsform. Dem Fachmann ist bekannt, dass "10-23 DNAzyme" mit vergleichbarer biologischer Aktivität mit abgewandelter katalytischer Domäne erhalten werden können.

In einer besonders bevorzugten Ausführung sind die Substratbindungsdomänen vollständig komplementär zu der Region, die die Spaltstelle flankiert. Um die Ziel RNA zu binden und sie zu spalten, muss das DNAzym jedoch nicht unbedingt vollständig komplementär sein. DNAzyme des 10-23 Typs spalten die Ziel mRNA an Purin-Pyrimidin Abfolge-Sequenzen. Im Sinne der vorliegenden Erfindung umfassen die DNAzyme vorzugsweise die in vivo aktiven DNAzyme gegen GATA-3 und Tbet gemäß der WO 2005/033314 A2, deren Inhalt zum Offenbarungsgehalt der vorliegenden Erfindung erklärt wird.

Ein Arzneimittel zur spezifischen Inhibition der GATA-3 Expression in vivo enthält insbesondere mindestens ein DNAzym ausgewählt aus der Gruppe bestehend aus DNAzymen mit einer Sequenz gemäß einer der Sequenzen SEQ ID NO 1 bis SEQ ID NO 70. Ein solches DNAzym bindet bevorzugt an eine mRNA, die für ein humanes GATA-3 Gen mit einer Gen-Sequenz ausgewählt aus den Sequenzen SEQ ID NO 151 (humanes GATA-3 aus Datenbank Nr.: XM_043124), SEQ ID NO 152 (humanes GATA-3 aus Datenbank Nr.: X58072) und SEQ ID NO 153 (humanes GATA-3, sequenziert aus Plasmid pCR2.1) kodiert.

Ein Arzneimittel zur spezifischen Inhibition der GATA-3 Expression in vivo enthält vorzugsweise das DNAzym hgd40 mit der Sequenz 5'-GTGGATGGAggctagctacaacgaGTCTTGGAG (SEQ ID NO 40).

Ein Arzneimittel zur spezifischen Inhibition der Tbet Expression in vivo enthält insbesondere mindestens ein DNAzym ausgewählt aus der Gruppe bestehend aus DNAzymen mit einer Sequenz gemäß einer der Sequenzen SEQ ID NO 71 bis SEQ ID NO 148. Ein solches DNAzym bindet bevorzugt an eine mRNA, die für ein humanes Tbet Gen mit einer Gen-Sequenz ausgewählt aus den Sequenzen SEQ ID NO 149 (humanes Tbet aus Datenbank Nr.: NM_013351) und SEQ ID NO 150 (humanes Tbet, sequenziert aus pBluescript-SK) kodiert.

Ein Arzneimittel zur spezifischen Inhibition der Tbet Expression in vivo enthält vorzugsweise das DNAzym td69 mit der Sequenz 5'-GGCAATGAAggctagctacaacgaTGGGTTTCT (SEQ ID NO 139) oder td70 mit der Sequenz 5'-TCACGGCAAggctagctacaacgaGAACTGGGT (SEQ ID NO 140).

Alternativ zu den DNAzymen kann das Arzneimittel zur spezifischen Inhibition der GATA-3 oder Tbet Expression eine geeignete siRNA enthalten.

Das Arzneimittel besitzt vorzugsweise eine Formulierung, die die genannten spezifischen Ribonukleinsäure- oder Desoxyribonukleinsäure-Moleküle den Patienten in Form einer pharmazeutisch akzeptablen Komposition entweder oral, rektal, parenteral, intravenös, intramuskulär oder subkutan, intracisternal, intravaginal, intraperitoneal, intrathekal, intravasculär, lokal (Puder, Salbe oder Tropfen) oder in Sprayform verabreicht werden kann. Dosierungsformen für die örtliche Administration des Arzneimittels dieser Erfindung schließen Salben, Puder, Sprays oder Inhalationsmittel ein. Die aktive Komponente wird unter sterilen Bedingungen mit einem physiologisch akzeptablen Trägerstoff und möglichen Preservativen, Puffern oder Treibmitteln, je nach Bedarf, vermischt.

Das Arzneimittel kann zur Therapie gegen sämtliche Erkrankungsgruppen, die mit chronischen Entzündungen einhergehen, angewendet werden.

Nach einer besonders bevorzugten Ausführung der vorliegenden Erfindung erfolgt die Behandlung von Patienten mit einem molekularen Phänotyp der Untergruppe "Th2 high" mit einem GATA-3 spezifischen DNAzym. Die Therapie eines Patienten mit einem molekularen Phänotyp der Untergruppe "Th1 high" erfolgt mit einem Tbet-spezifischen DNAzym. Weiterhin kann die Behandlung eines Patienten mit dem molekularen Phänotyp der Untergruppe "Th2 low" mit einem Tbet-spezifischen DNAzym und die Behandlung eines Patienten mit dem molekularen Phänotyp der Untergruppe "Th1 low" mit einem GATA-3 DNAzym erfolgen.

Ein erfindungsgemäßes Arzneimittel mit einem GATA-3 spezifischen DNAzym ist somit vorzugsweise für die Behandlung eines Patienten mit molekularem Phänotyp der Untergruppe "Th2 high" vorgesehen und ein Arzneimittel mit einem Tbet spezifischen DNAzym ist vorzugsweise für die Behandlung eines Patienten mit molekularem Phänotyp der Untergruppe "Th1 high" vorgesehen.

Erfindungsgemäß besteht ein besonderer Vorteil des Arzneimittels nach einer oben genannten Ausführungsform zur spezifischen Therapie einer erfindungsgemäß diagnostizierten Untergruppe an Patienten - nämlich "Th2 high", "Th2 low", "Th1 high" oder "Th1 low" - darin, dass mit Hilfe des spezifischen Arzneimittels, insbesondere eines DNAzyms und/oder einer siRNA, eine funktionelle Inaktivierung von gerade den kodierenden Ribonukleinsäure-Molekülen von Transkriptionsfaktoren erfolgt, deren differenzielle Expression vorher festgestellt wurde und die an der Entstehung der chronischen Entzündungsreaktionen und Autoimmunerkrankungen beteiligt sind. Diese Strategie unterscheidet sich deutlich gegenüber konventionellen Vorgehensweisen und auch gegenüber dem genannten Ansatz nach Corren et al., 2011, da dort einerseits beispielsweise die Menge an Periostin (Surrogatmarker) gemessen wird, dann aber eine Therapie gegen ein anderes Target, wie beispielsweise gegen IL-13 mit Hilfe eines Anti-IL-13 Antikörpers, vorgeschlagen wurde. Das erfindungsgemäße Arzneimittel hingegen weist eine hohe Spezifität auf. Es bewirkt eine Zell-spezifische Intervention und ist spezifisch für Kompartimente und Organe.

Darreichungsformen der erfindungsgemäßen Arzneimittels umfassen pharmazeutisch akzeptable Kompositionen, die Modifikationen und "Prodrugs" beinhalten, sofern sie nach zuverlässiger medizinischer Beurteilung keine übermäßige Toxizität, Irritationen oder allergische Reaktionen am Patienten auslösen. Der Terminus "Prodrug" bezieht sich auf Verbindungen, die zur Verbesserung der Aufnahme transformiert werden, wie beispielsweise durch Hydrolyse im Blut.

Das erfindungsgemäße Arzneimittel kann auch in der Form einer multiplen Emulsion zur Applikation der genannten spezifischen Nukleinsäuremoleküle eingesetzt werden. Eine geeignete multiple Emulsion umfasst dazu eine äußere Wasserphase W1, eine in der äußeren Wasserphase W1 dispergierte Ölphase O und eine in der Ölphase O dispergierte innere Wasserphase W2, wobei in der inneren Wasserphase W2 wenigstens ein Elektrolyt ausgewählt aus der Gruppe der Alkali- und Erdalkalihalogenide und -sulfate und wenigstens ein spezifisches Ribonukleinsäure- oder Desoxyribonukleinsäure-Molekül, vorzugsweise ein für GATA-3 oder Tbet spezifisches DNAzym, vorgesehen ist, wobei die äußere Wasserphase W1 einen hydrophilen Emulgator enthält, der ein Polymer von Ethylenoxid und Propylenoxid ist, und die Ölphase O von Triacylglyceriden gebildet ist und einen lipophilen Emulgator aus der Gruppe der Dimethicone aufweist. Mit Hilfe einer solchen multiplen Emulsion können insbesondere Nukleinsäuremoleküle besonders wirkungsvoll vor unerwünschtem Abbau geschützt werden.

Die Art der Dosierung wird vom behandelnden Arzt entsprechend den klinischen Faktoren bestimmt. Es ist dem Fachmann bekannt, dass die Art der Dosierung von verschiedenen Faktoren wie z.B. Körpergröße, Gewicht, Körperoberfläche, Alter, Geschlecht oder der allgemeinen Gesundheit des Patienten abhängig ist, aber auch von dem speziell zu verabreichenden Mittel, der Dauer und Art der Verabreichung und von anderen Medikamenten, die möglicherweise parallel verabreicht werden. Dabei kann nach einer besonders vorteilhaften Ausführungsform, die Menge des wirksamen Bestandteils des Arzneimittels an das gemessene Expressionsniveau angepasst werden. So kann bei erfolgter Einordnung in die Untergruppe "Th2 high" und einer festgestellten sehr hohen GATA-3 Genexpression eine erhöhte Dosis des wirksamen Bestandteils, insbesondere eines für GATA-3 spezifischen DNAzyms, verabreicht werden. Entsprechend kann bei erfolgter Einordnung in die Untergruppe "Th1 high" und einer festgestellten sehr hohen Tbet Genexpression eine erhöhte Dosis des wirksamen Bestandteils, insbesondere eines für Tbet spezifischen DNAzyms, verabreicht werden.

Ein weiterer Aspekt der vorliegenden Erfindung betrifft ein Kit zur Diagnose eines molekularen Phänotyps eines an einer Erkrankung, die mit chronischen Entzündungen einhergeht, leidenden humanen Patienten, wobei das Kit mindestens eine spezifische Komponente zur quantitativen Bestimmung der Protein oder mRNA-Menge von GATA-3 und/oder Tbet in einem biologischen Isolat des Patienten enthält.

Der erfindungsgemäße Kit zur Diagnose kann leicht in Form eines gebrauchsfertigen "Kits" dargeboten werden, welcher Antikörper oder Antigen, das an eine Oberfläche eines Trägers adsorbiert ist, umfasst und eine Zubereitung von humanen IgG-Antikörpern, die z.B. im Falle eines Menschen, eine Zubereitung von anti-human IgG-Antikörpern, die so markiert sind, dass sie durch eine Kaskade von Reaktionen des Typs Biotin-Streptavidin-Peroxidase oder -Alkalische Phosphatase nachgewiesen werden.

Alternativ umfasst der Kit zur Diagnose neben einen Träger auch Puffer und Reagenzien z.B. Reagenzien, die für den Nachweis der Reaktion erforderlich sind wie z.B. Streptavidin, das mit einem Marker gekoppelt ist, der eine Farbreaktion ergibt.

Alternativ umfasst der Kit zusätzlich eine Standardprobe von GATA-3 und/oder T-Bet zum Kalibrieren des Kits, wobei zum Nachweis der Protein oder mRNA-Menge von GATA-3 und/oder Tbet eine Standardprobe eingesetzt wird.

Bei einer bevorzugten Ausführungsform ist für die quantitative Bestimmung der Protein-Menge ein jeweils spezifischer Antikörper gegen GATA-3 und/oder Tbet enthalten. Gegebenenfalls können gemäß einer Abwandlung weitere Komponenten zur Ausführung eines Immunoassays, insbesondere eines ELISAs, enthalten sein.

Die weitere Komponente zur Durchführung des ELISAs ist ausgewählt aus der Gruppe, bestehend aus Lysepuffer zum Zellaufschluss, einer Mikrotiterplatte, Protein-Mengenstandards für GATA-3 und/oder Tbet, sekundäre Antikörper und ein gekoppeltes Enzym zur Umsetzung eines Substrats zur Detektion. Bevorzugt ist neben einem ersten spezifischen Antikörper gegen GATA-3- und/oder Tbet ein weiterer spezifischer Antikörper gegen GATA-3- oder Tbet vom Kit umfasst.

Das Kit kann für die quantitative Bestimmung der mRNA-Menge eine jeweils sequenzspezifische Sonde und /oder Primer für die GATA-3 und/oder Tbet Gene enthalten.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: den Einfluss von verschiedenen Detergenzien auf die Freisetzung von GATA-3 aus stimulierten Jurkat-Zellen,
- Fig. 2 a,b: zeigen Ergebnisse einer Quantifizierung von Tbet und GATA-3 mittels chromogenem Sandwich-ELISA,
- Fig. 3: eine Standardkurve eines GATA-3 ELISAs zur Quantifizierung von Proben
- Fig. 4: eine Standardkurve eines Tbet ELISAs zur Quantifizierung von Proben,
- Fig. 5: eine normierte Bestimmung von Tbet in Lysaten humaner peripherer mononukleärer Zellen (PBMC) von gesunden Probanden und Allergikern,
- Fig. 6: eine signifikante Verbesserung der allergischen Atemwegsentzündung nach viertägiger Behandlung mit dem GATA-3-spezifischen DNAzym hgd40 (SEQ ID NO 40) im Vergleich zu unbehandelten Mäusen,
- Fig. 7: den Einfluss des GATA-3-spezifischen DNAzyms hgd40 (SEQ ID NO 40) auf die Anzahl der in der chronischen Entzündung auftretenden Neutrophilen, die Anzahl der Eosinophilen in der BAL und die Freisetzung von IL-5 nach achtwöchiger Behandlung und
- Fig. 8: den Einfluss des GATA-3-spezifischen DNAzyms hgd40 (SEQ ID NO 40) auf die peribronchiale/perivaskuläre Inflammation und Goblet-zell Hyperplasie im Lungengewebe.

### Material und Methoden:

Zellen können zum Beispiel mittels Technologien isoliert werden, die auf der Bindung spezifischer Antikörper basieren. Hier werden Magnetic Beads, erhältlich von den Firmen Miltenyi (Macs-System), Dynal (DynaBeads) oder BD-Bioscience (iMAG) angewendet. Alternativ erfolgt dies über eine Zeltreinigung mittels Fluoreszenz-markierter Antikörper an Zellsortern beispielsweise der Firma Cytomation (MOFLO) oder BD-Bioscience (FACS-Vantage). Die Reinheit der Zielzellen ist bevorzugt bei mindestens 80%, stärker bevorzugt bei mindestens 95% und am meisten bevorzugt bei mindestens 99%.

Verfahren zur Isolierung der RNA sind z.B. in Sambrook and Russell, Molecular Cloning, A Laboratory Manual, 3. Auflage, Cold Spring Harbor Laboratory (2001), New York und Ausubel et al., Current Protocols in Molecular Biology, John Wiley & Sons (1998), New York, beschrieben. Außerdem ist es dem Durchschnittsfachmann möglich, kommerziell verfügbare Kits (Silika-Technologie) z.B. das RNeasy Kit von der Firma Qiagen, zur RNA Isolierung zu verwenden. Weiterhin ist es bevorzugt, direkt mRNA aus den Zielzellen durch Verwendung kommerzieller Kits beispielsweise der Firmen Qiagen (Oligotex mRNA Kit), Promega (PolyATract mRNA Isolation System) oder Miltenyi (mRNAdirect) zu reinigen.

### Ausführungsbeispiele

### Ausführungsbeispiel 1

GATA-3 und Tbet sind Proteine, die als so genannte Transkriptionsfaktoren ihre Wirkung im Zellkern von T-Helferzellen des Subtyps Th1 und Th2 entfalten. Um die Konzentration dieser beiden nukleären Proteine in einem bestimmten Volumen eines biologischen Isolats, insbesondere in einem bestimmten Volumen an Vollblut, zu bestimmen, müssen Zellen, welche GATA-3 und Tbet bilden, zunächst isoliert und danach lysiert werden. Ein direkter Nachweis dieser Proteine aus menschlichem Serum oder Plasma ist nicht möglich, da sie dort nicht in detektierbarer Konzentration vorhanden sind. Eine Analyse von GATA-3 und Tbet erfolgt daher in 4 Stufen:
- Abtrennung und Isolierung der GATA-3/Tbet exprimierenden Zellen von den übrigen zellulären Bestandteilen des Vollblutes
- Aufschluss der Zellen und Freisetzung der intrazellulären/nukleären Proteine
- Messung der Konzentration von GATA-3 und Tbet
- Normierung der gefundenen Konzentrationen von GATA-3 und Tbet

### Abtrennung und Isolierung der GATA-3/Tbet exprimierenden Zellen von den übrigen zellulären Bestandteilen des Vollblutes

Dies ist durch verschiedene Verfahren unterschiedlicher Komplexität durchführbar, insbesondere sind folgende Schritte zur Abtrennung und Isolierung im Sinne der vorliegenden Erfindung:
- eine Isolierung von Leukozyten aus Vollblut mittels Ficoll-Dichtegradienten-Zentrifugation mit anschließender Affinitätsreinigung der Th1/Th2 Zelltypen durch Antikörper gegen spezifische Oberflächenmarker,
- gegebenenfalls kann die Affinitätsreinigung der Th1/Th2 Zelltypen durch Antikörper gegen spezifische Oberflächenmarker auch als 1-stufiges Verfahren ohne vorherige Anreicherung der Leukozyten durchgeführt werden,
- gegebenenfalls reicht auch die Isolierung von Leukozyten durch Ficoll-Dichtegradienten-Zentrifugation aus Vollblut aus, um eine Quantifizierung der Proteine GATA-3 und Tbet durchzuführen,
- gegebenenfalls kann anstelle der Ficoll-Dichtegradienten-Zentrifugation auch eine bead-basierende Affinitätsreinigung der Th1/Th2 Zelltypen durch Antikörper gegen spezifische Oberflächenmarker in einer Deep-Well Platte im 96-well Format eingesetzt werden,
- bei Bedarf kann anstelle der Ficoll-Dichtegradienten-Zentrifugation auch eine bead-basierende Affinitätsreinigung der Leukozyten durch Antikörper gegen spezifische Oberflächenmarker, beispielsweise in einer Deep-Well Platte im 96-well Format, eingesetzt werden
- gegebenenfalls kann zur Gewinnung einer Leukozytenpräparation eine hypoosmolare Lyse der Erythrozyten erfolgen oder
- gegebenenfalls kann der Proteinaufschluss auch direkt aus Vollblut erfolgen.

### Der Aufschluss der Zellen und die Freisetzung der intrazellulären/nukleären Proteine

Dies kann durch unterschiedliche Verfahren und Prinzipien erreicht werden, insbesondere sind folgende Verfahrensschritte im Sinne der vorliegenden Erfindung:
- Zerstörung zellulärer Membranen durch Lysepuffer mit unterschiedlichen Wirkprinzipien:
   a) Hypotone Puffer, die ein Platzen der Zellen bewirken
   b) Detergenzhaltige Puffer, welche die Zellmembran zerstören und dadurch intrazelluläre Proteine freisetzen
   c) Puffer hoher lonenstärke bzw. osmotisch aktive Puffer, welche den Zellen Wasser entziehen und dadurch die Zellintegrität zerstören
- Physikalische Verfahren wie Erhitzen, Schockgefrieren oder Ultraschall
- Mechanische Verfahren wie Homogenisieren oder Mörsern.

Beispiele für detergenzhaltige Puffer könnten sein:
- Puffersysteme mit hoher Konzentration an ionischen (z.B. SDS oder Cholat und dessen Derivate) oder nicht-ionischen (z.B Triton oder Tween-20) Detergenzien
- Gemische aus ionischen und nicht-ionischen Detergenzien (z.B. Ripa-Puffer mit 50 mM Tris · HCl (pH 7,5), 150 mM NaCl, 1 % NP40, 0,5 % Natriumdeoxycholat und 0,1 % SDS)
- kommerziell erhältliche Lysepuffer mit unbekannter Zusammensetzung (z.B. M-PER)

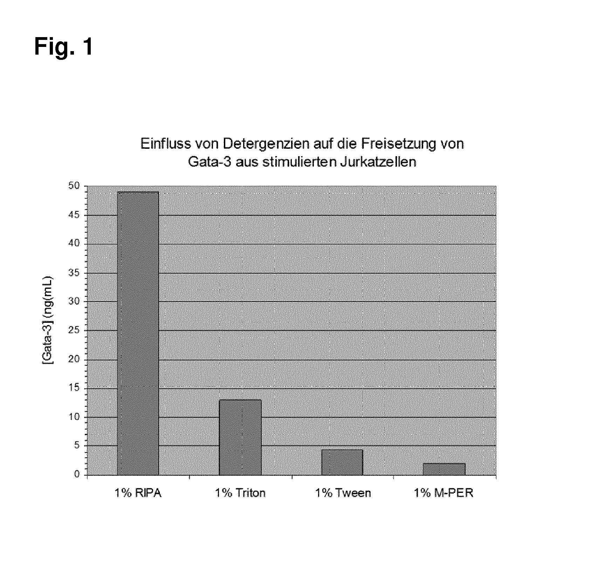

Der Einfluss von verschiedenen Detergenzien auf die Freisetzung von GATA-3 aus stimulierten Jurkat-Zellen ist in Fig. 1 dargestellt. Die Lyse von Jurkatzellen (humane T-Zell-Linie) durch verschiedene Lysepuffer und Quantifizierung von GATA-3 mittels ELISA ergab dabei eine besonders hohe Freisetzung von GATA-3 Protein bei Verwendung des RIPA Puffers (1% RIPA). Es wurden etwa 50 ng/ml GATA-3 nachgewiesen.

### Messung der Konzentration von GATA-3 und Tbet

Die Konzentration der beiden Transkriptionsfaktoren GATA-3 und Tbet kann prinzipiell mit verschiedenen Verfahren bestimmt werden. Im Sinne der vorliegenden Erfindung sind unter anderem:
- ELISA (enzyme-linked immunosorbent assay)
- CLIA (chemoluminescence-linked Immunosorbent assay)
- FLIA (fluorescence-linked Immunosorbent assay)
- Massenspektrometrische Verfahren
- Chromatographische Verfahren (z.B. Gaschromatographie)
- Flüssigkeitsbasierende Verfahren mit Festphasentrennung (z.B. HPLC)
- Mikro- und Nanofluidische Verfahren

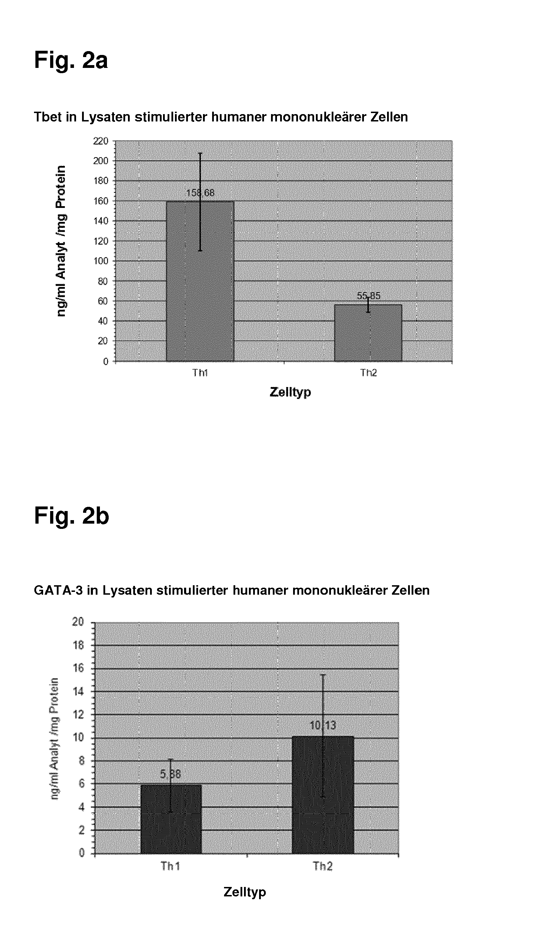

Die Fig. 2a und Fig. 2b zeigen Ergebnisse einer Quantifizierung von Tbet und GATA-3 mittels chromogenem Sandwich-ELISA. Die Zellen wurden durch Ficoll-Dichtegradienten-Zentrifugation aus Vollblut gewonnen. Die Zellen (stimulierte humane mononukleäre Zellen) wurden mit einem Ripa-Puffer lysiert. Das Lysat wurde mit zwei ELISA-Prototypverfahren hinsichtlich der Konzentration von GATA-3 und Tbet untersucht. Die Konzentration der beiden Proteine wurde in Bezug zur Gesamt-Proteinkonzentration der Zelllysate dargestellt (Normierung auf Proteingehalt).

Die Ergebnisse gemäß Fig. 2a zeigen, dass Th1-Zellen einen höheren Gehalt an Tbet (ca. 160 ng/ml Analyt/mg Protein) haben als Th2-Zellen (ca. 56 ng/ml Analyt/mg Protein) und sich dieser Umstand eindeutig aus dem Ergebnis des ELISA-Tests ablesen lässt:

| Zellen | ng/ml Tbet / mg Protein |
|---|---|
| Th2 B11-14 | 51,01 |
| Th2 B11-15 | 58,40 |
| Th2 B11-16 | 50,24 |
| Th2 B11-17 | 68,79 |
| Th2 B11-19 | 49,63 |
| Th2 B11-20 | 55,85 |
| Mean | 55,65 |
| STABW | 7,31 |
| VK(%) | 13,14 |
| | |
| Th1 B11-14 | 202,24 |
| Th1 B11-19 | 106,34 |
| Th1 B11-20 | 167,46 |
| Mean | 158,68 |
| STABW | 48,55 |
| VK(%) | 30,59 |

Der Tbet-Gehalt in den Th1-Zellen ist also hier mehr als zweifach, nämlich etwa um den Faktor drei, höher als in Th2-Zellen.

Gemäß Fig. 2b ist der Gehalt an GATA-3 in Th2-Zellen (ca. 10 ng/ml Analyt/mg Protein) höher ist als in Th1-Zellen (ca. 6 ng/ml Analyt/mg Protein). Der GATA-3-Gehalt ist in den Th2-Zellen also hier mehr als 1,5-fach - nämlich etwa um den Faktor 1,7 - höher als in Th1-Zellen.

Weiterhin kann man hier aus den Fig. 2a und 2b erkennen, dass sich bei Normierung auf den Proteingehalt das Mengenverhältnis von Tbet : GATA-3 in Th1-Zellen wesentlich von dem entsprechenden Verhältnis in Th2-Zellen unterscheidet. So beträgt das Mengenverhältnis von Tbet : GATA-3 hier in Th1-Zellen etwa 27, also mehr als 20, wohingegen das entsprechende Mengenverhältnis in Th2-Zellen ca. 6 beträgt, also weniger als 10.

Die quantitative Bestimmung von GATA-3 und Tbet erfolgt jeweils mittels eines Sandwich-ELISA (Enzyme-linked Immuno Sorbent Assay).

### Ausführungsbeispiel 2 - GATA-3 ELISA:

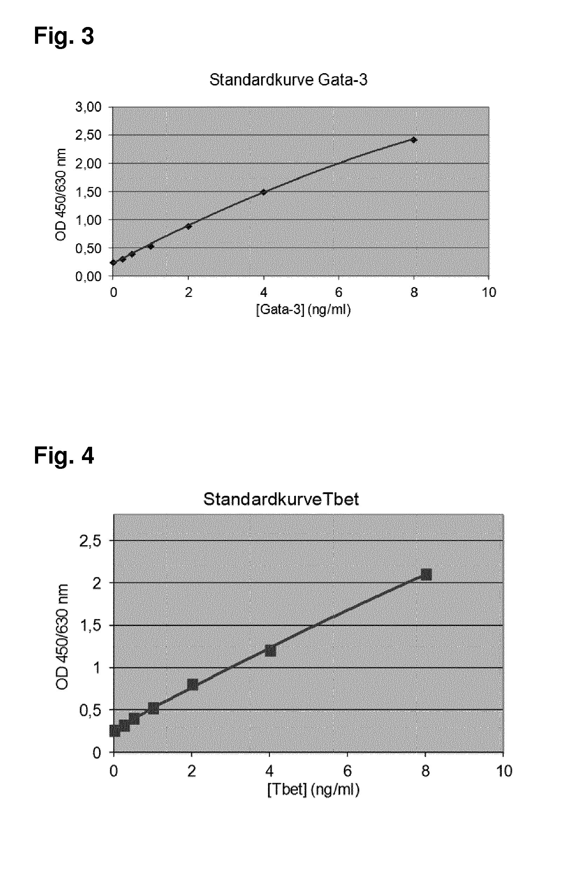

Die wells einer 96 well Mikrotiterplatte sind dazu mit spezifischen Antikörpern gegen GATA-3 beschichtet. Nach Zugabe der Probe bzw. eines Standards bindet GATA-3 an die Antikörper auf der 96-well Platte. Nach einem Waschschritt zur Entfernung der nicht gebundenen Substanzen erfolgt die Zugabe eines zweiten, spezifischen biotinylierten Antikörpers gegen GATA-3. Nach einem weiteren Waschschritt zur Entfernung der nicht gebundenen Substanzen wird Peroxidase-markiertes Streptavidin zugegeben. Nach einem letzten Waschschritt zur Entfernung der nicht gebundenen Substanzen wird Substrat zugegeben. Die Farbentwicklung wird nach einer definierten Zeit durch Zugabe einer Stopplösung beendet. Die Intensität der Farbentwicklung wird durch ein Mikrotiter-Plattenlesegerät quantifiziert. Die Quantifizierung der Proben erfolgt durch einen Vergleich mit den mitgeführten Standards bekannter Proteinkonzentration. Figur 3 zeigt eine entsprechende Standardkurve eines GATA-3 ELISAs.

Nach dem Ausführungsbeispiel für die Durchführung des GATA-3 ELISA betreffen die Schritte im Einzelnen:
- Anzahl der benötigten wells in einen Rahmen der 96-well Platte einsetzen
- Zugabe von 50 µl/well Assay Buffer
- Zugabe von 100 µl/well Standard / Kontrolle /Probe
- Inkubation für 60 Minuten auf dem Schüttler
- Alle wells 4 x mit je 400 µl/well Waschpuffer waschen
- Zugabe von 100 µl/well biotinyliertem anti-GATA-3 Antikörper
- Inkubation für 60 Minuten auf dem Schüttler
- Alle wells 4 x mit 400 µl/well Waschpuffer waschen
- Zugabe von 100µ/well Peroxidase-markiertem Streptavidin
- Inkubation für 30 Minuten auf dem Schüttler
- Alle wells 4 x mit 400 µl/well Waschpuffer waschen
- Zugabe von 100 µl/well Substrat
- Inkubation für 30 Minuten
- Stopp der Reaktion durch Zugabe von 100 µl Stopplösung
- Messung der Optischen Dichte bei 450 nm mit einem Mikrotiter-Plattenlesegerät

### Ausführungsbeispiel 3 - Tbet ELISA:

Der Nachweis des Tbet Proteins wird nach folgendem Testprinzip ausgeführt: Die quantitative Bestimmung von Tbet erfolgt mittels eines Sandwich-ELISA (Enzyme-linked Immuno Sorbent Assay). Die wells einer 96 well Mikrotiterplatte werden dazu mit spezifischen Antikörpern gegen Tbet beschichtet. Nach Zugabe der Probe bzw. des Standards bindet Tbet an die Antikörper auf der 96-well Platte. Nach einem Waschschritt zur Entfernung der nicht gebundenen Substanzen erfolgt die Zugabe eines zweiten spezifischen Antikörpers gegen Tbet. Nach einem weiteren Waschschritt zur Entfernung der nicht gebundenen Substanzen wird ein Peroxidase-markierter Antikörper gegen den Tbet-spezifischen Antikörper zugegeben. Nach einem letzten Waschschritt zur Entfernung der nicht gebundenen Substanzen wird Substrat zugegeben. Die Farbentwicklung wird nach einer definierten Zeit durch Zugabe einer Stopplösung beendet. Die Intensität der Farbentwicklung wird durch ein Mikrotiter-Plattenlesegerät quantifiziert. Die Quantifizierung der Proben erfolgt durch einen Vergleich mit den mitgeführten Standards bekannter Proteinkonzentration. Figur 4 zeigt eine entsprechende Standardkurve eines Tbet ELISAs.

Nach einem Ausführungsbeispiel für die Durchführung des Tbet ELISA betreffen die Schritte im Einzelnen:
- Anzahl der benötigten wells in einen Rahmen der 96-well Platte einsetzen
- Zugabe von 50 µl/well Assay Buffer
- Zugabe von 100 µl/well Standard / Kontrolle /Probe
- Inkubation für 60 Minuten auf dem Schüttler
- Alle wells 4 x mit je 400 µl/well Waschpuffer waschen
- Zugabe von 100 µl/well anti-Tbet Antikörper
- Inkubation für 60 Minuten auf dem Schüttler
- Alle wells 4 x mit 400 µl/well Waschpuffer waschen
- Zugabe von 100µ/well Peroxidase-markiertem anti-Tbet-spezifischer Antiköper
- Inkubation für 30 Minuten auf dem Schüttler
- Alle wells 4 x mit 400 µl/well Waschpuffer waschen
- Zugabe von 100 µl/well Substrat
- Inkubation für 30 Minuten
- Stopp der Reaktion durch Zugabe von 100 µl Stopplösung
- Messung der Optischen Dichte bei 450 nm mit einem Mikrotiter-Plattenlesegerät

### Normierung der Konzentrationen von GATA-3 und Tbet

Um Unterschiede in der Probenvorbereitung zu berücksichtigen, kann eine Normierung der Konzentrationen von GATA-3 und Tbet durchgeführt werden. Unterschiede in der Probenvorbereitung können z.B. entstehen durch:
- unterschiedliche Zellzahlen, die lysiert werden
- unterschiedliche Lyseeffizienzen der einzelnen Proben oder
- unterschiedlichen Gehalt an den verschiedenen Zelltypen innerhalb der Zellpräparationen.

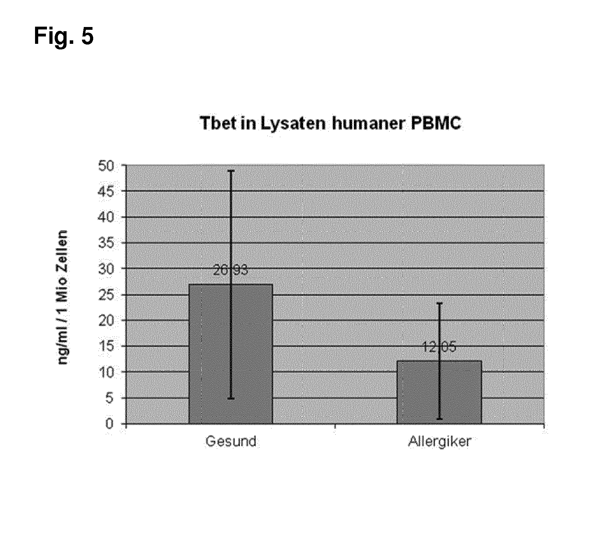

Möglichkeiten der Normierung sind erfindungsgemäß unter anderem:
- Normierung auf den Gesamt-Proteingehalt des Zelllysates (siehe unter "Messung der Konzentration von GATA-3 und Tbet")
- Normierung auf die Zellzahl, die lysiert wurde (siehe Fig. 5) oder
- Normierung auf die Konzentration spezifischer Markerproteine, die spezifisch in bestimmten Zelltypen gefunden werden.

Fig. 5. zeigt eine normierte Bestimmung von Tbet in Lysaten humaner peripherer mononukleärer Zellen (PBMC). Dabei erfolgte eine Lyse von PBMCs von gesunden Probanden und an allergischen Erkrankungen leidenden Patienten, wie z.B. allergisches Asthma Bronchiale, Rhinokonjunktivitis, allergische Sinusitis, atopische Dermatitis, Nahrungsmittelallergien. Die Konzentration von Tbet wurde auf die Zellzahl in den Lysaten normiert. Die Erkrankung ist Th2 Zellen abhängig und folgerichtig konnte bei den Allergikern eine geringere Konzentration an Tbet (ca. 12 ng/ml/1 Mio Zellen) im Vergleich zu gesunden Probenden (ca. 27 ng/ml/1 Mio Zellen) ermittelt werden. Die Tbet Konzentration war somit beim Allergiker etwas mehr als um den Faktor 2 im Vergleich zu einem gesunden Probanden vermindert. Eine Zuordnung der Patienten zum molekularen Phänotyp "Th1-low" ist hier somit einfach möglich, da die Tbet Genexpression im biologischen Isolat niedriger ist als ein definierter Referenzwert, hier die Tbet Genexpression von gesunden Probenden.

### Ausführungsbeispiel 4

In Abwandlung von Beispiel 2 und 3 wird nach Beispiel 4 zur Probenvorbereitung Th1/Th2 Zellen mittels magnetischer beads, die mit zellspezifischen Antikörpern beschichtet sind, angereichert. Anschließend erfolgte der Nachweis von GATA-3 gemäß der Vorschrift nach Beispiel 2.

### Ausführungsbeispiel 5

In Abwandlung von Beispiel 2 und 3 werden nach Beispiel 4 zur Probenvorbereitung Leukozyten mittels Größenausschluss-Filtration angereichert. Anschließend erfolgte der Nachweis von GATA-3 gemäß der Vorschrift nach Beispiel 2.

### Ausführungsbeispiel 6

Ein GATA-3-spezifisches DNAzym zeigt therapeutische Effekte im Mausmodell der OVAinduzierten allergischen Atemwegsentzündung vom "Th2-high"-Phänotyp.

Um den klinischen Phänotyp "Th2 high" bestmöglich im Mausmodell abzubilden, wurden BALB/c-Mäuse mit dem Modellallergen Ovalbumin (OVA) in Gegenwart des Adjuvanz Al(OH)₃ an den Tagen 0, 14 und 21 durch intraperitoneale Injektion sensibilisiert. An den Tagen 24-26 inhalierten die Mäuse ein 1%iges OVA-Aerosol, um eine Th2-dominierte allergische Entzündungsreaktion in der Lunge hervorzurufen. An den Tagen 23-26 wurde das GATA-3-spezifische DNAzym hgd40 (SEQ ID NO 40), gelöst in PBS, intranasal appliziert.

Dabei ist der Balb/c-Mausstamm dadurch charakterisiert, dass er präferentiell starke Th2-Antworten generiert. Dies wird verstärkt durch die Verwendung von AL(OH)₃ als Adjuvanz, dass die Ausbildung Th2-domierter Immunantworten deutlich unterstützt. Das beschriebene Mausmodell ist entsprechend gekennzeichnet durch eine massive Infiltration von Eosinophilen und Th2-Zellen in die Atemwege einhergehend mit einer Hyperplasie der mukusbildenden Goblet-Zellen mit verstärkter Mukusproduktion sowie Ausbildung einer Atemwegshyperreagibilität. Immunologisch sind neben allergenspezifischen Th2-Zellen, charakterisiert durch die Produktion der typischen Zytokine IL-4, IL-5 und IL-13, auch OVA-spezifische Antikörper der Immunglobulinklassen IgE und IgG1 (in der Maus beide Th2-abhängig) feststellbar. Alle diese Parameter sind typische klinische Merkmale eines "Th2-high" Phänotyps (Wenzel et al., Am J Respir Crit Care Med. 1999 Sep; 160(3):1001-8; Woodruff et al., 2009). Dabei ist die Reaktionsstärke hinsichtlich einiger Parameter im Tiermodell sogar noch deutlich stärker ausgeprägt als in der klinischen Situation beim humanen Patienten, so stellen z.B. eosinophile Granulozyten ca. 60-70 % aller Leukozyten in der bronchoalveolären Lavage (BAL) im Mausmodell dar, während bereits 3-5% dieser Zellen im Sputum von Patienten auf einen Th2-dominierten Phänotyp hinweisen.

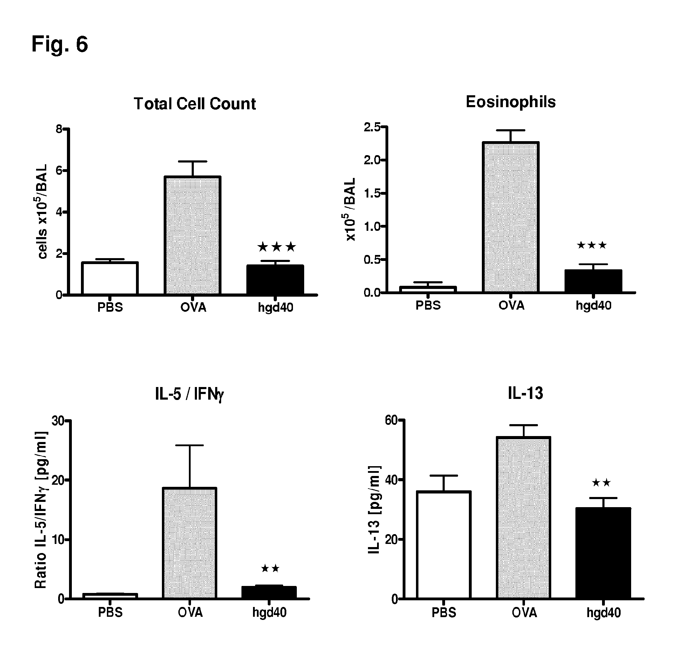

Gemäß Fig. 6 konnte nach viertägiger Behandlung mit dem GATA-3-spezifischen DNAzym hgd40 (SEQ ID NO 40) im Vergleich zu unbehandelten Mäusen eine signifikante Verbesserung der allergischen Atemwegsentzündung festgestellt werden. Vor allem die Anzahl von Eosinophilen in der BAL wurde erheblich reduziert. Desweiteren konnten die BAL-Konzentrationen der für den Phänotyp "Th2 high" charakteristischen Zytokine IL-5 und IL-13 deutlich reduziert werden.

### Ausführungsbeispiel 7

Ein GATA-3-spezifisches DNAzym zeigt signifikante therapeutische Effekte im chronischen Mausmodelleiner Th2-dominierten allergischen Atemwegsentzündung

Um den klinischen Phänotyp "Th2 high" bestmöglich auch in einem chronischen Mausmodell abzubilden, wurden BALB/c-Mäuse mit Ovalbumin (OVA) in Gegenwart des Adjuvanz Al(OH)₃ an den Tagen 0, 14 und 21 durch intraperitoneale Injektion sensibilisiert. Mittels zweimal wöchentlicher OVA-Aerosol-Provokationen über einen Zeitraum von 14 Wochen wurde in den Mäusen eine chronische Entzündung der Atemwege hervorgerufen. Während der letzten acht Wochen wurde entweder mit Budesonid oder dem GATA-3-spezifischem DNAzym hdg40 durch intranasale Applikation dreimal pro Woche therapiert (bis Tag 121).

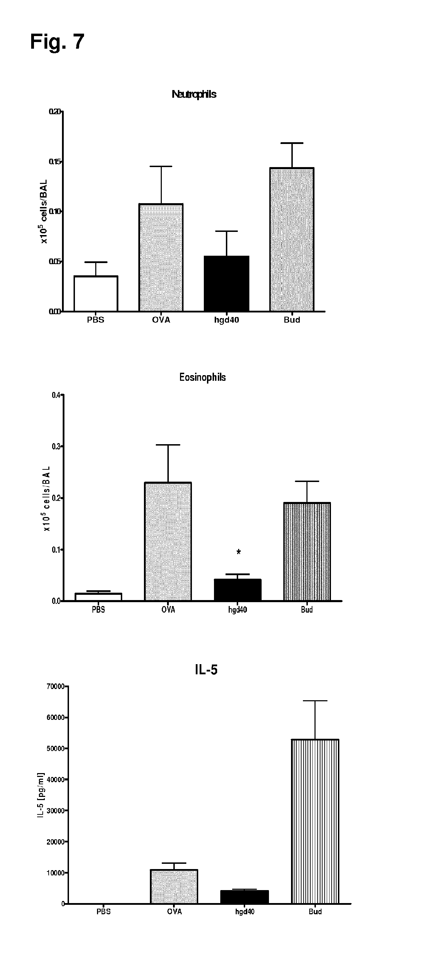

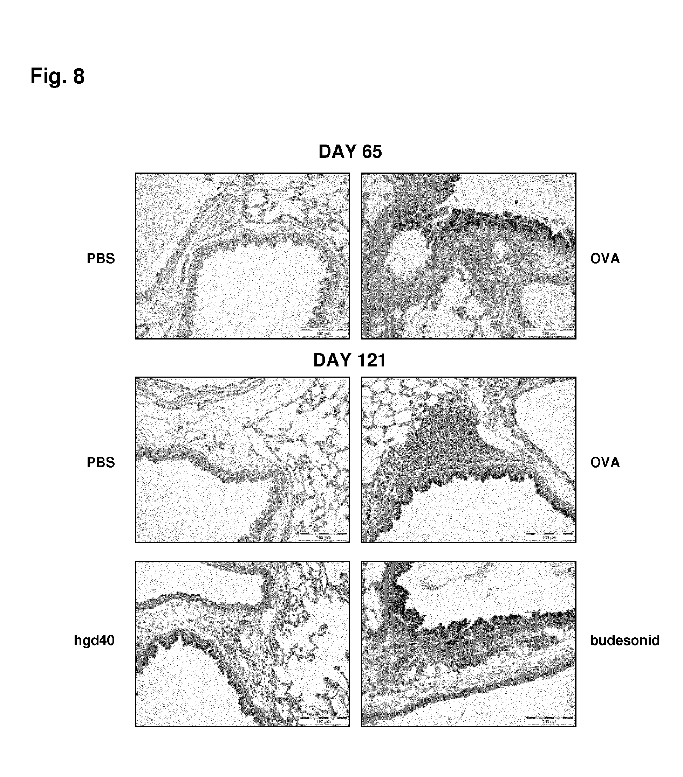

Gemäß Fig. 7 und 8 konnte nach achtwöchiger Behandlung mit dem GATA-3-spezifischen DNAzym hgd40 (SEQ ID NO 40) die Anzahl der Eosinophilen in der BAL signifikant reduziert werden und zudem eine Reduzierung der Anzahl der in der chronischen Entzündung auftretenden Neutrophilen festgestellt werden. Einhergehend damit wurden im Lungengewebe eine verminderte peribronchiale/perivaskuläre Inflammation und reduzierte Goblet-zell Hyperplasie festgestellt. Gleichzeitig wurden in restimulierten Lymphzyten der mit hgd40 Behandelten eine verminderte Freisetzung von IL-5 beobachtet. In der Budesonid-Gruppe hingegen konnte keine signifikante Verbesserung der Parameter festgestellt werden.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Verfahren zur Diagnose eines molekularen Phänotyps eines an einer Erkrankung, die mit chronischen Entzündungen einhergeht, leidenden humanen Patienten, wobei der molekulare Phänotyp ausgewählt ist aus der Gruppe bestehend aus den Untergruppen "Th2 high", "Th2 low", "Th1 high" und "Th1 low" und die Genexpression von GATA-3 und/oder Tbet in einem biologischen Isolat des Patienten gemessen und für die Zuordnung zu einem molekularen Phänotyp der Erkrankung genutzt wird.

2. Verfahren nach Anspruch 1, **dadurch gekennzeichnet, dass**
- das Expressionsniveau von GATA-3 und/oder Tbet über die Proteinmenge bestimmt wird, wobei die Protein-Menge insbesondere mit Hilfe eines Immunassays, vorzugsweise eines ELISA-Tests, eines Radioimmunassays, eines Elektrochemilumineszenz-Immunassays, eines CLIA (chemoluminescence-linked Immunosorbent assay), eines FLIA (fluorescence-linked Immunosorbent assay) oder eines Multiplex-Assays, quantitativ bestimmt wird; und/oder
- das Expressionsniveau von GATA-3 und/oder Tbet über die mRNA-Menge bestimmt wird, wobei die mRNA-Menge insbesondere mit Hilfe einer PCR oder eines Micro-Array-Chips quantitativ bestimmt wird.

3. Verfahren nach einem der Ansprüche 1 oder 2, **dadurch gekennzeichnet, dass** eine Zuordnung des Patienten zu einem molekularen Phänotyp der Untergruppe "Th2 high" erfolgt, wenn mindestens eine der folgenden Bedingungen erfüllt ist:
a) die GATA-3 Genexpression im biologischen Isolat ist höher als ein definierter Referenzwert,
b) das Verhältnis von GATA-3 : Tbet Genexpression im biologischen Isolat ist höher als ein definierter Referenzwert.

4. Verfahren nach einem der Ansprüche 1 oder 2, **dadurch gekennzeichnet, dass** eine Zuordnung des Patienten zu einem molekularen Phänotyp der Untergruppe "Th2 low" erfolgt, wenn mindestens eine der folgenden Bedingungen erfüllt ist:
a) die GATA-3 Genexpression im biologischen Isolat ist niedriger als ein definierter Referenzwert,
b) das Verhältnis von GATA-3 : Tbet Genexpression im biologischen Isolat ist niedriger als ein definierter Referenzwert.

5. Verfahren nach einem der Ansprüche 1 oder 2, **dadurch gekennzeichnet, dass** eine Zuordnung des Patienten zu einem molekularen Phänotyp der Untergruppe "Th1 high" erfolgt, wenn mindestens eine der folgenden Bedingungen erfüllt ist:
a) die Tbet Genexpression im biologischen Isolat ist höher als ein definierter Referenzwert,
b) das Verhältnis von Tbet : GATA-3 Genexpression im biologischen Isolat ist höher als ein definierter Referenzwert.

6. Verfahren nach einem der Ansprüche 1 oder 2, **dadurch gekennzeichnet, dass** eine Zuordnung des Patienten zu einem molekularen Phänotyp der Untergruppe "Th1 low" erfolgt, wenn mindestens eine der folgenden Bedingungen erfüllt ist:
a) die Tbet Genexpression im biologischen Isolat ist niedriger als ein definierter Referenzwert,
b) das Verhältnis von Tbet : GATA-3 Genexpression im biologischen Isolat ist niedriger als ein definierter Referenzwert.

7. Verfahren nach einem der Ansprüche 1 bis 6, **dadurch gekennzeichnet, dass** neben der Bestimmung der Genexpression von GATA-3 und/oder Tbet eine Bestimmung des Serum-lgE-Spiegels und/oder der Zahl der eosinophilen Granulocyten erfolgt und/oder der Fe_{NO}-Wert ermittelt wird.

8. Verfahren nach einem der Ansprüche 1 bis 7, **dadurch gekennzeichnet, dass** die mit chronischen Entzündungen einhergehende Erkrankung Th2-bedingt, insbesondere allergisches Asthma Bronchiale, Rhinokonjunktivitis, allergische Sinusitis, atopische Dermatitis, Nahrungsmittelallergien, Pemphigus, Ulcerative Colitis, parasitäre Erkrankungen, oder Th1-bedingt, insbesondere Psoriasis, allergisches Kontaktekzem, Morbus Crohn, COPD, Rheumatoide Arthritis, Autoimmunerkrankungen, Typ 1 Diabetes mellitus oder MS, ist.

9. Arzneimittel zur Behandlung eines an einer Erkrankung, die mit chronischen Entzündungen einhergeht, leidenden humanen Patienten mit einem molekularen Phänotyp, wobei der molekulare Phänotyp ausgewählt ist aus der Gruppe bestehend aus den Untergruppen "Th2 high", "Th2 low", "Th1 high" und "Th1 low" und gemäß einem Verfahren nach einem der Ansprüche 1 bis 8 ermittelt wurde.

10. Arzneimittel nach Anspruch 9, **dadurch gekennzeichnet, dass** ein für GATA-3 oder Tbet spezifisches DNAzym enthalten ist.

11. Arzneimittel nach Anspruch 10, **dadurch gekennzeichnet, dass** das Arzneimittel mit einem GATA-3 spezifischen DNAzym für die Behandlung eines Patienten mit molekularem Phänotyp der Untergruppe "Th2 high" vorgesehen ist oder das Arzneimittel mit einem Tbet spezifischen DNAzym für die Behandlung eines Patienten mit molekularem Phänotyp der Untergruppe "Th1 high" vorgesehen ist.

12. Arzneimittel nach Anspruch 10 oder 11, **dadurch gekennzeichnet, dass** das Arzneimittel eine multiple Emulsion umfasst, welche eine äußere Wasserphase W1, eine in der äußeren Wasserphase W1 dispergierte Ölphase O und eine in der Ölphase O dispergierte innere Wasserphase W2 aufweist, wobei in der inneren Wasserphase W2 wenigstens ein Elektrolyt ausgewählt aus der Gruppe der Alkali- und Erdalkalihalogenide und -sulfate und wenigstens ein für GATA-3 oder Tbet spezifisches DNAzym vorgesehen ist, wobei die äußere Wasserphase W1 einen hydrophilen Emulgator enthält, der ein Polymer von Ethylenoxid und Propylenoxid ist, und die Ölphase O von Triacylglyceriden gebildet ist und einen lipophilen Emulgator aus der Gruppe der Dimethicone aufweist.

13. Kit zur Diagnose eines molekularen Phänotyps eines an einer mit chronischen Entzündungen einhergehenden Erkrankung leidenden humanen Patienten, wobei das Kit mindestens eine spezifische Komponente zur quantitativen Bestimmung der Protein- und/oder mRNA-Menge von GATA-3 und/oder Tbet in einem biologischen Isolat des Patienten gemäß einem Verfahren nach einem der Ansprüche 1 bis 8 enthält.

14. Kit nach Anspruch 13, **dadurch gekennzeichnet, dass** für die quantitative Bestimmung der Protein-Menge ein jeweils spezifischer Antikörper gegen GATA-3 und/oder Tbet enthalten ist, wobei vorzugsweise Komponenten zur Ausführung eines Immunassays, insbesondere eines ELISAs, enthalten sind.

15. Kit nach Anspruch 13, **dadurch gekennzeichnet, dass** für die quantitative Bestimmung der mRNA-Menge eine sequenzspezifische Sonde und/oder ein sequenzspezifischer Primer für GATA-3 und/oder Tbet Gene enthalten ist/sind.
